(19) Europäisches Patentamt
European Patent Office
Office européen des brevets

(11) Publication number: **0 279 263 B1**

# (12) EUROPEAN PATENT SPECIFICATION

(45) Date of publication of patent specification: **04.08.93**

(51) Int. Cl.5: **C07D 333/58**, C07D 307/81, C07D 209/14, A61K 31/38, A61K 31/34, A61K 31/40

(21) Application number: **88101386.6**

(22) Date of filing: **01.02.88**

## (54) Indole, benzofuran, benzothiophene containing lipoxygenase inhibiting compounds.

(30) Priority: **10.02.87 US 12970**
**10.06.87 US 60784**
**11.01.88 US 138073**

(43) Date of publication of application:
**24.08.88 Bulletin 88/34**

(45) Publication of the grant of the patent:
**04.08.93 Bulletin 93/31**

(84) Designated Contracting States:
**BE CH DE ES FR GB GR IT LI NL SE**

(56) References cited:
**DE-A- 1 813 836**

(73) Proprietor: **ABBOTT LABORATORIES**

**Abbott Park, Illinois 60064(US)**

(72) Inventor: **Summers, James B., Jr.**
**1108 Crestfield Avenue**
**Libertyville Illinois 60048(US)**
Inventor: **Gunn, Bruce P.**
**849 Lexington Lane**
**Island Lake Illinois 60042(US)**
Inventor: **Brooks, Dee W.**
**926 Bartlett Terrace**
**Libertyville Illinois 60048(US)**

(74) Representative: **Modiano, Guido et al**
**c/o Modiano & Associati S.r.l. Via Meravigli, 16**
**I-20123 Milano (IT)**

**EP 0 279 263 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to organic compounds which inhibit lipoxygenase enzymes. It also relates to compositions for inhibiting lipoxygenase enzymes in human and animal hosts in need of such treatment.

The lipoxygenases are a family of enzymes which catalyze the oxygenation of arachidonic acid. The enzyme 5-lipoxygenase converts arachidonic acid to 5-hydroperoxyeicosatetraenoic acid (5-HPETE). This is the first step in the metabolic pathway yielding 5-hydroxyeicosatetraenoic acid (5-HETE) and the important class of mediators, the leukotrienes (LTs).

Similarly, 12- and 15-lipoxygenase, convert arachidonic acid to 12- and 15-HPETE, respectively. Biochemical reduction of 12-HPETE leads to 12-HETE, while 15-HPETE is the precursor of the class of biological agents known as the lipoxins.

A variety of biological effects are associated with these products from lipoxygenase metabolism of arachidonic acid and they have been implicated as mediators in various disease states. For example, the LTs $C_4$ and $D_4$ are potent constrictors of human airways *in vitro*, and aerosol administration of these substances to non-asthmatic volunteers induces broncho-constriction. $LTB_4$ and 5-HETE are potent chemotactic factors for inflammatory cells such as polymorphonuclear leukocytes. They also have been found in the synovial fluid of rheumatoid arthritic patients. Leukotrienes have also been implicated as important mediators in asthma, rheumatoid arthritis, gout, psoriasis, allergic rhinitis, adult respiratory distress syndrome, Crohn's disease, endotoxin shock, inflammatory bowel disease and/or ischemia induced myocardial or brain injury, among others. The biological activity of the LTs has been reviewed by Lewis and Austen (*J. Clinical Invest.* **73,** 889, 1984) and by J. Sirois (*Adv. Lipid Res.* **21,** 78, 1985).

The product 12-HETE has been found in high levels in epidermal tissue of patients with psoriasis. The lipoxins have recently been shown to stimulate elastase and superoxide ion release from neutrophils.

Thus, lipoxygenase enzymes are believed to play an important role in the biosynthesis of mediators of asthma, allergy, arthritis, psoriasis, and inflammation. It is postulated that interrupting the biochemical pathways involved in the various manifestations of these disease states will provide effective systemic and/or symptomatic treatment of these diseases.

In accordance with the present invention there are 5- and/or 12-lipoxygenase inhibiting compounds of the formula:

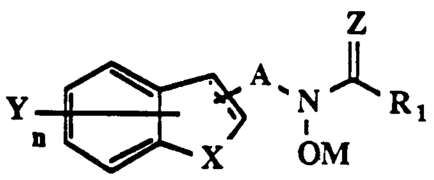

Formula I

wherein $R_1$ is (1) hydrogen, (2) $C_1$ to $C_4$ alkyl, (3) $C_2$ to $C_4$ alkenyl, or (4) $NR_2R_3$, wherein $R_2$ and $R_3$ are independently selected from (1) hydrogen, (2) $C_1$ to $C_4$ alkyl and (3) hydroxyl, but $R_2$ and $R_3$ are not simultaneously hydroxyl;

wherein X is oxygen, sulfur, $SO_2$, or $NR_4$, wherein $R_4$ is (1) hydrogen, (2) $C_1$ to $C_6$ alkyl, (3) $C_1$ to $C_6$ alkoyl, (4) aroyl, or (5) alkylsulfonyl;

A is selected from $C_1$ to $C_6$ alkylene $C_2$ to $C_6$ alkenylene:

n is 1-5;

Y is selected independently at each occurrence from (1) hydrogen, (2) halogen, (3) hydroxy, (4) cyano, (5) halosubstituted alkyl, (6) $C_1$ to $C_{12}$ alkyl, (7) $C_2$ to $C_{12}$ alkenyl, (8) $C_1$ to $C_{12}$ alkoxy, (9) $C_3$ to $C_8$ cycloalkyl, (10) $C_1$ to $C_8$ thioalkyl, (11) aryl, (12) aryloxy, (13) aroyl, (14) arylalkyl in which the alkyl portion is of from 1 to 12 carbon atoms, (15) arylalkenyl in which the alkyl portion is of from 2 to 12 carbon atoms, (16) arylalkoxy in which the alkoxy portion is of from 1 to 12 carbon atoms, (17) arylthioalkoxy in which the alkoxy portion is of from 1 to 12 carbon atoms, and substituted derivatives of (18) aryl, (19) aryloxy, (20) aroyl, (21) arylalkyl in which the alkyl is of from 1 to 12 carbon atoms, (22) arylalkenyl in which the alkenyl portion is of from 2 to 12 carbon atoms, (23) arylalkoxy in which the alkoxy portion is of from 1 to 12 carbon atoms, or (24) arylthioalkoxy in which the alkoxy portion is of from 1 to 12 carbon atoms, wherein substituents are selected from halo, nitro, cyano, $C_1$ to $C_{12}$ alkyl, $C_1$ to $C_{12}$ alkoxy, and $C_1$ to $C_{12}$ halosubstituted alkyl;

Z is oxygen or sulfur,

and M is hydrogen, a pharmaceutically acceptable cation, aroyl, or $C_1$ to $C_{12}$ alkoyl.

The dotted line within the five membered ring of formula I signifies that a single or double bond are to be selected from. The substituent(s) Y and the linking group A may be attached at any available position on either ring. The term "aryl" refers to both carbocyclic and heterocyclic aromatic radicals.

The preferred compounds of the present invention are of formula II:

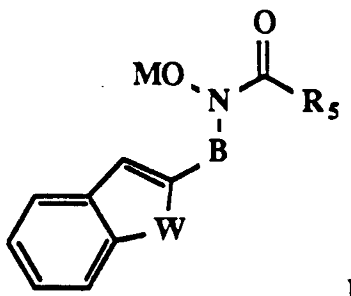

Formula II

In these preferred compounds $R_5$ is $C_1$ or $C_2$ alkyl, or $NR_6R_7$ where $R_6$ and $R_7$ are independently selected from hydrogen and $C_1$ or $C_2$ alkyl; B is $CH_2$ or $CHCH_3$; W is oxygen or sulfur; and M is hydrogen, a pharmaceutically acceptable cation, aroyl, or $C_1$ to $C_{12}$ alkoyl.

Examples of compounds which are within the scope of the present invention include the following:

N-hydroxy-N-(1-benzo[b]thien-2-ylethyl)acetamide
N-hydroxy-N-(1-benzo[b]thien-2-ylethyl)urea
N-hydroxy-N-(1-benzo[b]thien-2-ylethyl)N'-methyl urea
N-hydroxy-N-(1-benzo[b]thien-2-ylethyl)N',N'-dimethyl urea
N-hydroxy-N-benzo[b]thien-2-ylmethyl urea
N-hydroxy-N-benzo[b]thien-2-ylmethyl N'-methyl urea
N-hydroxy-N-benzo[b]thien-2-ylmethyl N',N'-dimethyl urea
N-hydroxy-N-(1-benzo[b]thien-3-ylethyl) acetamide
N-hydroxy-N-(1-benzo[b]thien-3-ylethyl)urea
N-hydroxy-N-[1-(3-methylbenzo[b]thien-2-yl)ethyl] urea
N-hydroxy-N-[1-(5-(2,2-dimethylethyl)benzo[b]thien-3-yl)ethyl]urea
N-hydroxy-N-[1-benzo[b]thien-2-ylethyl) acetamide 1,1-dioxide
N-hydroxy-N-(1-benzo[b]thien-2-ylethyl) urea 1,1-dioxide
N-hydroxy-N-(1-benzo[b]fur-2-ylethyl) acetamide
N-hydroxy-N-(1-benzo[b]fur-2-ylethyl) urea
N-hydroxy-N-(1-(1-methylindol-3-yl)ethyl) acetamide
N-hydroxy-N-(1-(1-methylindol-3-yl)ethyl) urea
N-hydroxy-N-(1-(1-methylindol-3-yl)ethyl) N'-methyl urea
N-hydroxy-N-(1-benzo[b]thien-2-yl)ethyl) urea sodium salt
N-hydroxy-N-(1-benzo[b]thien-2-ylethyl) formamide
N-hydroxy-N-(1-benzo[b]thien-2-ylethyl) 2-methylpropionamide
N-hydroxy-N-[1-(5-chlorobenzo[b]fur-2-yl)ethyl] urea
N-hydroxy-N-[1-(5-methoxybenzo[b]fur-2-yl)ethyl] urea
N-hydroxy-N-(1-(1-methylindol-2-yl)ethyl) urea
N-hyxoxy-N-(1-benzo[b]thien-2-ylethyl) thiourea
N-hydroxy-N-[1-(3-thioethylbenzo[b]thien-2-yl)ethyl] urea
N-hydroxy-N-[1-(5-fluorobenzo[b]thien-2-yl)ethyl] urea
N-hydroxy-N-(2-benzo[b]thien-2-yl-1-methylethyl) urea
N-hydroxy-N-(3-benzo[b]thien-2-ylprop-2-enyl) acetamide
N-hydroxy-N-(3-benzo[b]thien-2-ylprop-2-enyl) urea
N-hydroxy-N-[1-(5,7-dichlorobenzo[b]fur-2-yl)ethyl] urea
N-hydroxy-N-[1-(7-methoxybenzo[b]fur-2-yl)ethyl] urea
N-hydroxy-N-[1-(7-methoxybenzo[b]fur-2-yl)ethyl] N'-methyl urea
N-hydroxy-N-[1-(7-methoxybenzo[b]fur-2-yl)ethyl] urea
N-hydroxy-N-(1-indol-2-ylethyl) N'-methyl urea
N-hydroxy-N-[1-(5-chloroindol-2-yl)ethyl] N'-methyl urea
N-hydroxy-N-[1-(1-acetylindol-2-yl)ethyl] urea

N-hydroxy-N-[1-(1-methanesulfonylindol-2-yl)ethyl] urea
N-hydroxy-N-benzo[b]thien-7-ylmethyl urea
N-hydroxy-N-[1-(2,3-dihydrobenzo[b]fur-yl)ethyl] urea
N,N'-dihydroxy-N-(1-benzo[b]thien-2-ylethyl) urea
N-hydroxy-N-(1-benzo[b]thien-2-ylethyl) N'-ethylurea
N-hydroxy-N-(1-benzo[b]thien-2-ylethyl) N'-methyl thiourea
N-hydroxy-N-benzo[b]thien-2-ylmethyl-N'-ethyl urea
N-hydroxy-N-(1-benzo[b]thien-2-yl)-3-methylpropyl urea
N-hydroxy-N-benzo[b]fur-2-ylmethyl urea
N-hydroxy-N-benzo[b]fur-2-ylmethyl N'-methyl urea
N-hydroxy-N-[1-(6-phenylmethoxybenzo[b]fur-2-yl)ethyl] urea
N-hydroxy-N-[1-(6-phenylmethoxybenzo[b]fur-2-yl)ethyl] N'-methyl urea
N-hydroxy-N-(1-indol-2-yl)ethyl) urea
N-hydroxy-N-[1-(3-hydroxybenzo[b]thien-2-yl)ethyl] urea
N-hydroxy-N-[1-(5-trifluoromethylbenzo[b]thien-2-yl)ethyl] urea
N-hydroxy-N-[1-(7-methoxybenzo[b]thien-2-yl)ethyl] urea
N-hydroxy-N-[1-(5-phenylbenzo[b]thien-2-yl)ethyl] urea
N-hydroxy-N-[1-(5-phenylmethoxy-benzo[b]thien-2-yl)ethyl] urea
N-hydroxy-N-[1-(2-benzo[b]thien-2-yl)propyl] urea
N-hydroxy-N-(1-benzo[b]thien-2-ylethyl) propionamide
N-hydroxy-N-(1-benzo[b]thien-2-ylethyl) propenamide
N-hydroxy-N-indol-2-ylmethyl acetamide
N-hydroxy-N-(1-benzo[b]thien-3-ylethyl) acetamide
N-hydroxy-N-[1-(5-fluorobenzo[b]fur-2-yl)ethyl] acetamide
N-hydroxy-N-(1-(5-phenoxybenzo[b]fur-2-yl)ethyl) acetamide
N-hydroxy-N-[1-(5-(4-fluorophenyl)methyl)benzo[b]thien-2-yl)ethyl] acetamide
N-hydroxy-N-(1-benzo[b]thien-2-ylethyl) urea potassium salt
N-hydroxy-N-(1-benzo[b]thien-2-ylethyl) acetamide ammonium salt
N-hydroxy-N-(1-benzo[b]thien-2-ylethyl) acetamide triethyl ammonium salt
N-hydroxy-N-(1-benzo[b]thien-2-ylethyl) acetamide tetraethyl ammonium salt
N-butyryloxy-N-(1-benzo[b]thien-2-ylethyl) urea
N-benzoyloxy-N-(1-benzo[b]thienylethyl) urea

The term "alkylene" is used herein to mean straight or branched chain spacer radicals such as $-CH_2-$, $-CHCH_3-$, $-C(CH_3)_2-$, $-CH(C_2H_5)-$, $-CH_2CH_2-$, $-CH_2CHCH_3-$, $C(CH_3)_2C(CH_3)_2-$ and $CH_2CH_2CH_2$.

The term "alkenylene" is used herein to mean straight or branched chain unsaturated spacer radicals such as $-CH=CH-$, $-CH=CHCH_2-$, $CH=CHCH(CH_3)-$, $-C(CH_3)=CHCH_2-$, $-CH_2CH=CHCH_2-$ and $C(CH_3)_2CH=CHC(CH_3)_2-$.

The team "alkyl" is used herein to mean straight or branched chain radicals of 1 to 12 carbon atoms, including, but not limited to methyl, ethyl, n-propyl, isopropyl, n-butyl, sec-butyl, isobutyl and tert-butyl.

The term "alkenyl" is used herein to mean straight or branched chain unsaturated radicals of 2 to 12 carbon atoms, including, but not limited to ethenyl, 1-propenyl, 2-propenyl, 2-methyl-1-propenyl, 1-butenyl and 2-butenyl.

The term "cycloalkyl" is used herein to mean cyclic radicals, preferably of 3 to 8 carbons, including, but not limited to cyclopropyl, cyclobutyl, cyclopentyl and cyclohexyl.

The term "alkoxy" is used herein to mean $-OR_8$ wherein $R_8$ is an alkyl radical, including, but not limited to methoxy, ethoxy, isopropoxy, n-butoxy, sec-butoxy, isobutoxy and tert-butoxy.

The term "thioalkyl" is used herein to mean $-SR_9$ wherein $R_9$ is an alkyl radical, including, but not limited to thiomethyl, thioethyl, thioisopropyl, n-thiobutyl, sec-thiobutyl, isothiobutyl and tert-thiobutyl.

The term "alkoyl" is used herein to mean $-COR_{10}$ wherein $R_{10}$ is an alkyl radical, including, but not limited to formyl, acetyl, propionyl, butyryl, isobutyryl and pivaloyl.

The term "carboalkoxy" is used herein to mean $-COR_{11}$ wherein $R_{11}$ is an alkoxy radical, including, but not limited to carbomethoxy, carboethoxy, carboisopropoxy, carbobutoxy, carbosec-butoxy, carboiso-butoxy and carbotert-butoxy.

The term "aryl" is used herein to mean substituted and unsubstituted carbocyclic and heterocylic aromatic radicals wherein the substituents are chosen from halo, nitro, cyano, $C_1$ to $C_{12}$ alkyl, alkoxy and halosubstituted alkyl. including, but not limited to phenyl, 1- or 2-naphthyl, 2-, 3-, or 4-pyridyl and 2-, 3-furyl.

The term "aroyl" is used herein to mean $-COR_{12}$ wherein $R_{12}$ is an aryl radical, including, but not limited to benzoyl, 1-naphthoyl and 2-naphthoyl.

The term "aryloxy" is used herein to mean $-OR_{13}$ wherein $R_{13}$ is an aryl radical, including, but not limited to phenoxy, 1-naphthoxy and 2-naphthoxy.

The term "arylalkoxy" is used herein to mean $-OR_{14}$ wherein $R_{14}$ is an arylalkyl radical, including, but not limited to phenylmethoxy (i.e., benzyloxy), 4-fluorobenzyloxy, 1-phenylethoxy, 2-phenylethoxy, diphenylmethoxy, 1-naphthylmethoxy, 2-napthylmethoxy, 9-fluorenoxy, 2-, 3- or 4-pyridylmethoxy and 2-, 3-, 4-, 5-, 6-, 7-, 8-quinolylmethoxy.

The term "arylthioalkoxy" is used herein to mean $-SR_{15}$ wherein $R_{15}$ is an arylalkyl radical, including, but not limited to phenylthiomethoxy (i.e., thiobenzyloxy), 4-fluorothiobenzyloxy, 1-phenylthioethoxy, 2-phenylthioethoxy, diphenylthiomethoxy and 1-naphthylthiomethoxy.

The term "arylalkyl" is used herein to mean an aryl group appended to an alkyl radical, including, but not limited to phenylmethyl (benzyl), 1-phenylethyl, 2-phenylethyl, 1-naphthylethyl and 2-pyridylmethyl.

The term "arylalkenyl" is used herein to mean an aryl group appended to an alkenyl radical, including, but not limited to phenylethenyl, 3-phenylprop-1-enyl, 3-phenylprop-2-enyl and 1-naphthylethenyl.

The term "alkylsulfonyl" is used herein to mean $-SO_2R_{16}$ wherein $R_{16}$ is an alkyl radical, including, but not limited to methylsulfonyl (i.e. mesityl), ethyl sulfonyl and isopropylsulfonyl.

The terms "halo" and "halogen" are used herein to mean radicals derived from the elements fluorine, chlorine, bromine, or iodine.

The term "halosubstituted alkyl" refers to an alkyl radical as described above substituted with one or more halogens, including, but not limited to chloromethyl, trifluoromethyl and 2,2,2-trichloroethyl.

The term "pharmaceutically acceptable cation" refers to non-toxic cations including but not limited to cations based on the alkali and alkaline earth metals, such as sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine and ethylamine.

The term "lipoxygenase" is used herein to mean 5- and/or 12-lipoxygenase, the enzymes which oxidize arachidonic acid at the 5 and 12 positions. respectively.

The compounds of the invention inhibit lipoxygenase activity, which makes the compounds useful in the treatment and prevention of disease states in which lipoxygenase may be involved, including, but not limited to, asthma, rheumatoid arthritis, gout, psoriasis, allergic rhinitis, adult respiratory distress syndrome, Crohn's disease, endotoxin shock, inflammatory bowel disease and/or ischemia induced myocardial or brain injury. In some cases this will involve preventing the underlying cause of the disease state and in other cases, while the underlying disease will not be affected the compounds of this invention will have the benefit of ameliorating the symptoms or preventing the manifestations of the disease.

The compounds of the present invention may be administered orally, parenterally or topically in dosage unit formulations containing conventional nontoxic pharmaceutically acceptable carriers, adjuvants and vehicles as desired

The term parenteral as used herein includes subcutaneous, intravenous, intra-arterial injection or infusion techniques, without limitation. The term "topically" encompasses administration rectally and by inhalation spray, as well as by the more common routes of the skin and the mucous membranes of the mouth and nose.

Total daily dose of the compounds of this invention administered to a host in single or divided doses may be in amounts, for example, of from about 0.001 to about 100 mg/kg body weight daily and more usually 0.1 to 20 mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated

## Formulation of Pharmaceutical Composition

This invention also provides pharmaceutical compositions in unit dosage form for the inhibition of 5- or 12-lipoxygenase activity in a human or lower animal host in need of such treatment, comprising a compound of this invention and one or more nontoxic pharmaceutically acceptable carriers, adjuvants or vehicles. The amount of active ingredient that may be combined with such materials to produce a single dosage form will vary depending upon various factors, as indicated above.

A variety of materials can be used as carriers, adjuvants and vehicles in the composition of this invention, as available in the pharmaceutical arts. Injectable preparations, such as oleaginous solutions, suspensions or emulsions, may be formulated according to known art, using suitable dispersing or wetting

agents and suspending agents, as needed. The sterile injectable preparation may employ a nontoxic parenterally acceptable diluent or solvent as, for example, sterile nonpyrogenic water or 1,3-butanediol.

Among the other acceptable vehicles and solvents that may be employed are 5% dextrose injection, Ringer's injection and isotonic sodium chloride injection (as described in the USP/NF). In addition, sterile, fixed oils are conventionally employed as solvents or suspending media. For this purpose any bland fixed oil may be used, including synthetic mono-, di- or triglycerides. Fatty acids such as oleic acid can also be used in the preparation of injectable compositions.

Suppositories for rectal administration of the compound of this invention can be prepared by mixing the drug with suitable nonirritating excipient such as cocoa butter and polyethylene glycols, which are solid at ordinary temperatures but liquid at body temperature and which therefore melt in the rectum and release the drug.

Solid dosage forms for oral administration include capsules, tablets, pills, troches, lozenges, powders and granules. In such solid dosage forms, the active compound may be admixed with at least one inert diluent such as sucrose, lactose or starch. Such dosage forms may also comprise, as is normal practice, pharmaceutical adjuvant substances, e.g., stearate lubricating agents. In the case of capsules, tablets and pills, the dosage forms may also comprise buffering agents. Solid oral preparations can also be prepared with enteric or other coatings which modulate release of the active ingredients.

Liquid dosage forms for oral administration include pharmaceutically acceptable emulsions, solutions, suspensions, syrups and elixirs containing inert nontoxic diluents commonly used in the art, such as water and alcohol. Such compositions may also comprise adjuvants, such as wetting agents, emulsifying suspending, sweetening, flavoring and perfuming agents.

Synthesis of the Compounds

Several synthetic methods may be used to prepare compounds of this invention. Some of these methods are described by schemes 1-6 below. Although in each case the sequence is illustrated with a compound of formula I wherein $R_1$ is methyl or $NH_2$, A is $-CHCH_3-$ X is sulfur, Y is hydrogen, and Z is oxygen, it will be seen from the examples that other compounds of this invention can be prepared in the same manner using the appropriate stung materials. Compounds of formula I wherein $R_1$ is alkyl, alkenyl, $N(alkyl)_2$ or hydrogen may be prepared as described in scheme 1.

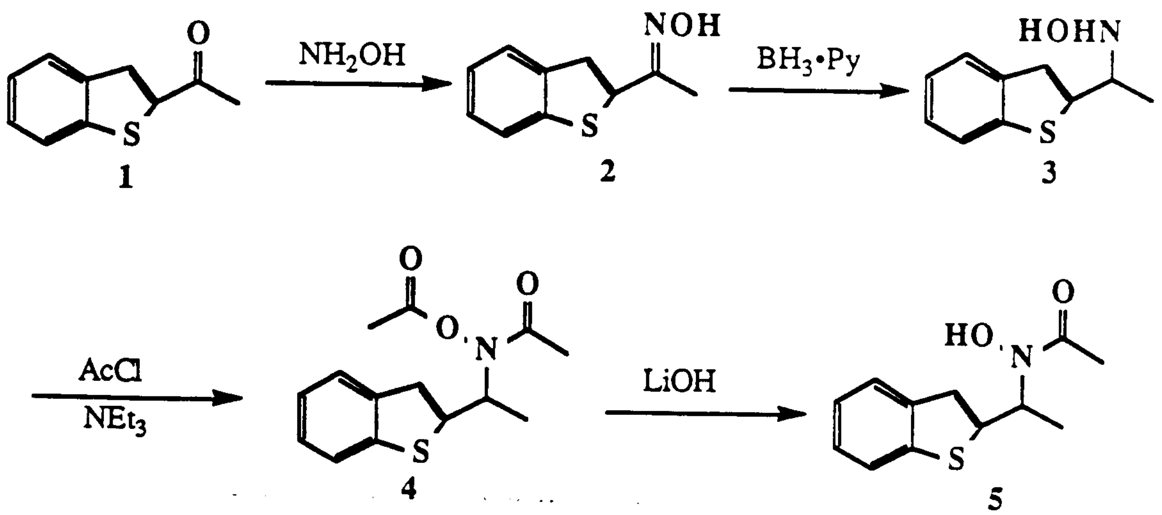

1 2 3
4 5

Scheme 1

In scheme 1, 2-acetyl benzo[b]thiophene, **1** is treated with hydroxylamine in ethanol/pyridine to produce the oxime **2**. This is reduced to the hydroxylamine **3** with borane pyridine complex and then converted to the N,O-diacetate **4** with acetyl chloride and triethylamine. The diacetate is converted to the hydroxamic acid **5** by hydrolysis with lithium hydroxide.

Other reagents may also be used to carry out the same transformation. For example **2** may be converted to **3** using borane trimethyl amine, borane-tetrahydrofuran, or other borane complexes. Intermediate **2** may also be converted to **3** with sodium cyanoborohydride or with phenyldimethylsilane in trifluoroacetic acid. Hydroxylamine **3** can also be converted to **4** with acylating agents such as acetic anhydride in the presence of other bases such as pyridine.

Compounds of formula I wherein $R_1$ is $NR_2R_3$ can be prepared according to the method outlined in scheme 2, below.

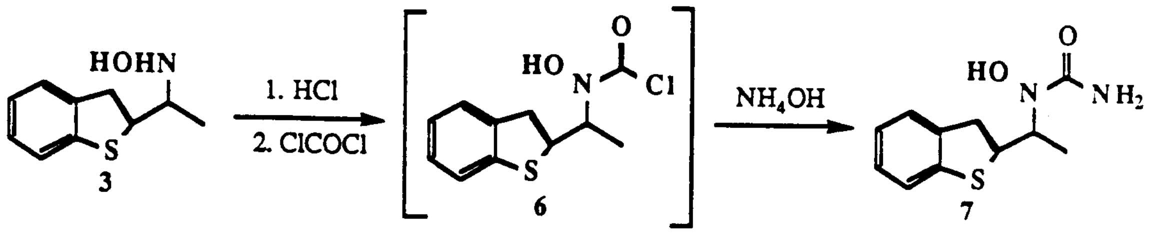


**3** **6** **7**

Scheme 2

Hydroxylamine **3**, the synthesis of which was described above, is treated with gaseous HC1 followed by phosgene. The resulting putative carbamoyl chloride **6** is reacted without isolation with aqueous ammonia to yield the urea **7**.

Compounds of formula I, wherein $R_1$ is $NR_2R_3$ and wherein at least one of either $R_2$ or $R_3$ is hydrogen can also be prepared according to Scheme 3, below.

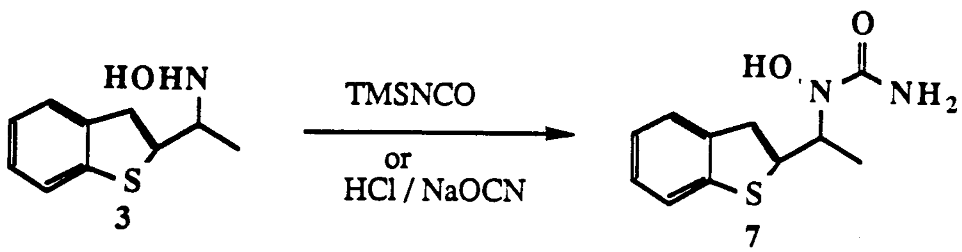


**3** **7**

Scheme 3

Hydroxylamine **3** is treated with trimethylsilyl isocyanate (TMSNCO), followed by ammonium chloride workup to give the urea **7**. Alternatively, **3** can be treated with sodium or potassium cyanate in an acidic solution to yield the urea **7**.

In addition to the methods described above, hydroxylamines such as **3** can be prepared as shown in scheme 4, below.

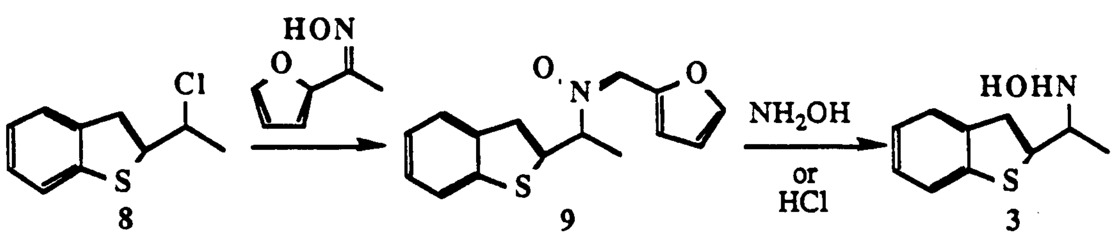


**8** **9** **3**

Scheme 4

Chloride **8** is treated with Z-furfuraldehyde oxime and a base such as sodium methoxide to give nitrone **9**. The nitrone is then hydrolyzed under acidic conditions or with hydroxylamine. The hydroxyl amine can be converted to compounds such as **5** and **7** using the methodology described above. Compounds with other leaving groups including bromides, iodides, tosylates, mesylates, triflates can be used instead of chloride **8**.

In addition to the methods described above, compounds of this invention may also be prepared as described in scheme 5, below.

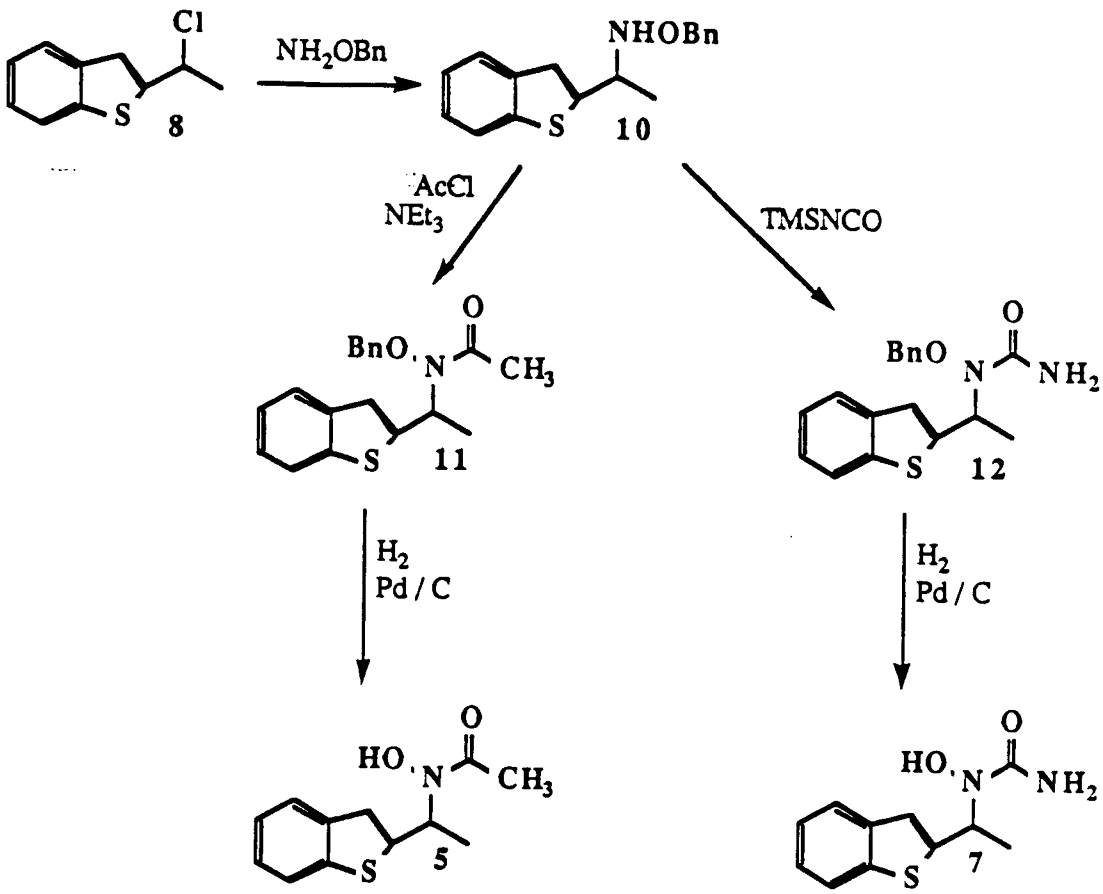


Scheme 5

Chloride **8** is heated with O-benzylhydroxylamine in a solvent such as dimethylsulfoxide or tetrahydrofuran to yield the new hydroxylamine **10**. This can either be reacted with acetyl chloride as in Scheme 1 to yield **11** or with trimethylsilyl isocyanate as in scheme 3 to yield **12**. Compounds **11** and **12** are then hydrogenated to yield **5** and **7** respectively. In addition **11** and **12** may converted to **5** and **7** by treatment with ethane thiol in the presence of aluminum trichloride.

Other O-protected hydroxylamines may also be used in place of O-benzylhydroxylamine such as O-tetrahydropyranyl hydroxylamine. Further, other methods may be used to convert **10** to **7**, such as treatment with phosgene followed by ammonium hydroxide such as described in scheme 2, or treatment with sodium cyanate as described in Scheme 3.

Compounds of this invention in which A is $-CH_2-$ or -CH(alkyl)- may also be prepared as described in Scheme 6.

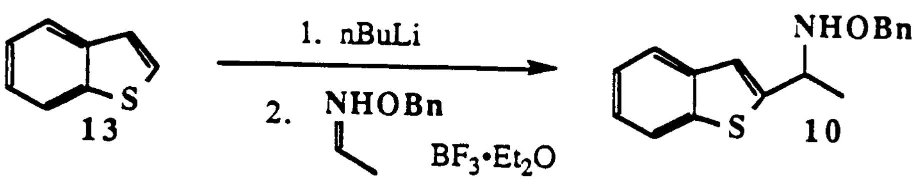


Scheme 6

Benzo[b]thiophene **13** is first converted to 2-lithiobenzo[b]thiophene by treatment with n-butyllithium. This is then treated with the O-benzyloxime of acetaldehyde in the presence of $BF_3 \cdot Et_2O$ to give O-benzylhydrox-

ylamine **10**. This may be converted to the compounds such as **5** or **7** as described in scheme 4. Other O-protected oximes can be substituted for the O-benzyl oxime.

The following examples further illustrate the synthesis and use of compounds of this invention. The appropriate designations for $R_1$, A, X and Y as defined by formula I are given for each example below. Unless otherwise noted, Z is oxygen and M is hydrogen.

Example 1

*N*-hydroxy-*N*-(1-benzo[b]thien-2-ylethyl) acetamide

a. 2-Acetyl benzo[b]thiophene. *Method a.* Using the method described in Scheme 1, benzo[b]thiophene (10 g, 75 mmole) was dissolved in THF (50 mL) and cooled to -78°C. Butyl lithium (28 mL, 2.7 M in hexanes) was added. The mixture was stirred for 15 minutes and *N,O*-dimethyl acetohydroxamic acid was added. Following an additional 30 minutes of stirring, the reaction was quenched at -78°C with ethanol and 2N HCl solution and extracted into ether. The solvent was removed *in vacuo* and the residue chromatographed on silica gel eluting with 20% ether in pentane to yield 6.9g of the desired product as a white solid.

*Method b.* To a solution of benzo[b]thiophene (10.0 g, 75 mmole) in THF (50 mL) was added n-butyl lithium (33 mL, 2.5M in hexanes) at -70°C under $N_2$ The mixture, containing a white precipitate, was stirred at -70°C for 1 hour. Acetaldehyde (4.6 mL, 82 mmole) was added dropwise. After a few minutes the reaction was quenched with saturated $NH_4Cl$ solution. The layers were separated, the organic layer dried over $MgSO_4$, filtered, and evaporated to give a white solid (10 g) which was used directly for the next step.

The alcohol prepared as described above (1.0 g) in acetone (50 mL) was cooled to 5°C and Jones Reagent was added dropwise until the orange yellow color persisted (1.4 mL). The reaction mixture was diluted with water and the desired product precipitated. It was collected by filtration to give 0.85 g.

b. 2-Acetyl benzo[b]thiophene oxime. 2-Acetyl benzo[b]thiophene (5 g, 28.4 mmole), prepared as described in step a above, and hydroxylamine hydrochioride (3.0 g, 42.6 mmole) were dissolved in a mixture of ethanol (50 mL) and pyridine (50 mL) and allowed to stir at room temperature for 2 hours. Most of the solvent was removed *in vacuo* and the residue dissolved in ether. After washing with 2N HCl (100 mL), the solution was dried over $MgSO_4$ and evaporated. A white crystalline solid was obtained and was carried on without further purification.

An alternative work-up may also be used. The reaction mixture was diluted with water (300 mL) and the product precipitated. It was filtered off and dried *in vacuo*.

c. 1-Benzo[b]thien-2-ylethyl hydroxylamine. The oxime prepared as in step b above (3.5 g, 18.5 mmole) was dissolved in ethanol (25 mL) and cooled to 0°C. Borane pyridine complex (3.7 mL, 37 mmole) was added *via* syringe under nitrogen followed ten minutes later by 20% HCl in ethanol (30 mL). Within thirty minutes the reaction was complete and was brought to pH 9 with the addition of solid sodium carbonate or 2N NaOH. The mixture was extracted into ether and dried over $MgSO_4$. After evaporation a white solid (3.0 g) was obtained. This was carried on without ether purification.

d. *N*-Acetoxy-*N*-(1-benzo[b]thien-2-ylethyl) acetamide. The hydroxylamine (1.0 g, 5.2 mmole) prepared as in step c above and pyridine (1.0 mL, 13 mmole) were dissolved in tetrahydrofuran (40 mL) and cooled to 0°C. Acetyl chloride (1.0 mL, 13 mmole) was added slowly. After stirring for 30 minutes the reaction mixture was washed with 2N HCl, dried over $MgSO_4$ and evaporated.

e. *N*-hydroxy-*N*-(1-benzo[b]thien-2-ylethyl) acetamide. The material obtained in the previous step (1.0 g) was dissolved in isopropyl alcohol (10 mL) and lithium hydroxide (1.0 g) in water (10 mL). After stiring for thirty minutes, most of the solvent was removed *in vacuo*. The residue was neutralized with 2N HCl, extracted with ether, and the organic phase was then dried over $MgSO_4$ and evaporated. The desired product was obtained as a white crystalline solid (750 mg) following silica gel chromatography. ($R_1 = CH_3$, $A = CHCH_3$, X = S, Y = H).

Melting Point: 108-110°C

NMR (300 MHz, DMSO-$d_6$): 1.56 (d, 3H); 2.02 (s, 3H); 5.90 (m, 1H); 7.29-7.38 (m, 3H); 7.75-7.92 (m, 2H); 9.75 (brs, 1H).

Mass spectrum (EI): 235 $M^+$, 218, 176, 161, 128.

Example 2

*N*-hydroxy-*N*-(1-benzo[b]thien-2-ylethyl) urea

Method a. Using the method of Scheme 3, 1-benzo[b]thien-2-yl ethyl hydroxyl amine prepared as described in example 1, step c (2.0 g, 10 mmole), was refluxed for thirty minutes with trimethylsilyl isocyanate (1.65, 14.2 mmole) in dioxane (30 mL). The reaction mixture was then washed with saturated $NH_4Cl$ solution, dried with MgSO4, and evaporated.

Method b. Using the method of Scheme 2, 1-benzo[b]thien-2-yl ethyl hydroxyl amine prepared as described in example 1, step c, was dissolved in toluene (100 ml) and HCl gas was bubbled through the mixture at a moderate rate for about four minutes. The solution was then heated to reflux and phosgene was bubbled through for another four minutes. After an additional one hour reflux, the mixture was allowed to cool to room temperature and then added to excess cold ammonium hydroxide solution. The precipitate was collected and recrystallized. ($R_1 = NH_2$, $A = CHCH_3$, X = S [2-isomer], Y = H)

Melting Point: 157-158 ° C.

NMR (300 MHz, DMSO-$d_6$): 1.51 (d, 3H); 5.55 (q, 1H); 6.45 (brs, 2H), 7.25-7.37 (m, 3H); 7.75-7.91 (m, 2H); 9.35 (s, 1H).

Mass spectrum (CI-$NH_3$): 237 $(M+1)^+$, 221, 194, 176, 161.

Example 3

*N*-hydroxy-*N*-(1-benzo[b]thien-2-ylethyl) *N'*-methyl urea

The desired compound was prepared according to the method of example 2, method a, except using methyl isocyanate instead of trimethylsilyl isocyanate. ($R_1 = NHCH_3$, $A = CHCH_3$, X = S [2-isomer], Y = H)

Melting Point: 149-150 ° C.

NMR (300 MHz, DMSO-$d_6$): 1.51 (d, 3H, J = 7.5 Hz); 2.60 (d, 3H); 5.55 (q, 1H, J = 7.5 Hz); 6.98 (m, 1H); 7.24-7.37 (m, 3H); 7.73-7.79 (m, 1H); 7.85-7.91 (m, 1H); 9.17 (s, 1H).

Mass Spectrum(CI-$NH_3$): 251 $(M+H)^+$, 268 $(N+NH_3)^+$

Example 4

*N*-hydroxy-*N*-(1-benzo[b]thien-2-ylethyl) *N'N'*-dimethyl urea

The desired compound was prepared according to the method of example 1, except using dimethylcarbamoyl chloride instead of acetyl chloride. ($R_1 = N(CH_3)_2$, $A = CHCH_3$, X = S [2-isomer], Y = H).

Melting Point: 139-141 ° C.

NMR (300 MHz, DMSO-$d_6$); 1.54 (d, 3H, J = 7.5 Hz); 2.87 (s, 6H); 5.24 (q, 1H); 7.25-7.37 (m, 3H); 7.74-7.79 (m, 1H); 7.85-7.91 (m, 1H); 8.92 (s, 1H).

Mass Spectrum (CI-$NH_3$): 264 $(M+H)^+$, 282 $(M+NH_4)^+$

Example 5

*N*-hydroxy-*N*-benzo[b]thien-2-ylmethyl urea

The desired compound was prepared according to the method of example 1, except using dimethyl formamide instead of ***N,O***-dimethyl acetohydroxamic acid. ($R_1 = NH_2$, $A = CH_2$, X = S [2-isomer], Y = H).

Melting Point: 170.5-172 ° C.

NMR (300 MHz, DMSO-$d_6$): 4.74 (s, 2H); 6.47 (brs, 2H); 7.23 (m, 2H); 7.78 (m, 1H); 7.90 (m, 1H); 9.53 (s, 1H)

Mass spectrum (CI-$NH_3$): 223 $(M+H)^+$, 240 $(M+NH_4)^+$

Example 6

*N*-hydroxy-*N*-benzo[b]thien-2-ylmethyl *N'*-methyl urea

The desired compound was prepared according to the method of example 1, except using dimethyl formamide instead of N,O-dimethyl acetohydroxamic acid and using methyl isocyanate instead of trimethyl-

silyl isocyanate. ($R_1$ = $NHCH_3$, A = $CH_2$, X = S [2-isomer], Y = H).

Melting Point: 160-161 °C.

NMR (300 MHz, DMSO-$d_6$): 2.61 (d, 3H); 4.74 (s, 2H); 7.01 (q, 1H); 7.26-7.38 (m, 3H); 7.74-7.97 (m, 2H); 9.46 (s, 1H).

Mass Spectrum (CI-$NH_3$): 237 $(M+H)^+$, 254 $(M+NH_4)^+$

Example 7

*N*-hydroxy-*N*-benzo[b]thien-2-ylmethyl *N'*,*N'*-dimethyl urea

The desired compound was prepared according to the method of example 1, except using dimethyl formamide instead of *N*,*O*-dimethyl acetohydroxamic acid and using dimethylcarbamoyl chloride instead of acetyl chloride. ($R_1$ = $N(CH_3)_2$, A = $CH_2$, X = S [2-isomer], Y = H).

Melting Point: 145-147 °C.

NMR (300 MHz, DMSO-$d_6$): 2.88 (s, 6H); 4.59 (s, 2H); 7.26-7.37 (m, 3H); 7.74-7.80 (m, 1H); 7.87-7.93 (m, 1H); 9.31 (s, 1H).

Mass Spectrum (CI-$NH_3$): 250 $(M+H)^+$, 268 $(M+NH_4)^+$

Example 8

*N*-hydroxy-*N*-(1-benzo[b]thien-3-ylethyl) acetamide

a. 3-Acetyl benzothiophene. A solution of benzothiophene (20 g, 149 mmole) in nitroethane (100 mL) was added to a solution of $AlCl_3$ (59.7 g, 448 mmole) and acetyl chloride (10.6 mL, 149 mmole) in nitroethane (150 mL) at 0 °C. The ice bath was removed after the addition was complete and the mixture was allowed to stir for 15 hours at room temperature. The mixture was poured onto ice (500 mL) and concentrated HCl (150 mL). The desired material was extracted into ethyl acetate which was dried over $MgSO_4$. The dark brown oil which resulted was chromatographed on silica gel eluting with 10% ethyl acetate in hexanes to give the desired material (3.8 g).

b. *N*-hydroxy-*N*-(1-benzo[b]thien-3-yl)ethyl acetamide wasprepared using the methods of example 1, steps b-e, except using the 3-acetyl benzo[b]thiophene instead of 2-acetylbenzo[b]thiophene. ($R_1$ = $CH_3$, A = $CHCH_3$, X = S [3-isomer], Y = H).

Melting Point: 123-125 °C

NMR (300 MHz, DMSO-$d_6$): 1.57 (d, 3H); 2.00 (brs, 3H); 6.03 (q, 1H), 7.32-7.44 (m, 2H); 7.71 (s, 1H); 7.80-8.02 (m, 2H); 9.45 (brs, 1H).

Mass Spectrum (CI-$NH_3$): 236 $(M+H)^+$, 253 $(M+NH_4)^+$.

Example 9

*N*-hydroxy-*N*-(1-benzo[b]thien-3-ylethyl) urea

The desired material was prepared according to the method of example 2, except using 3-acetyl benzo-[b]thiophene instead of 2-acetyl benzo[b]thiophene. ($R_1$ = $NH_2$, A = $CHCH_3$, X = S [3-isomer], Y = H).

Melting Point: 180 °C dec.

NMR (300 MHz, DMSO-$d_6$): 1.50 (d, 3H); 5.73 (q, 1H); 6.35 (br s, 2H); 7.30-7.43 (m, 2H); 7.67 (s, 1H); 7.90-8.00 (m, 2H); 8.96 (s, 1H).

Mass Spectrum (EI): 236 $M^+$, 219, 161, 128.

Example 10

*N*-hydroxy-*N*-[1-(3-methylbenzo[b]thien-2-yl)ethyl] urea

The desired compound was prepared according to the method of example 2, except using 3-methylbenzothiophene instead of benzothiophene. ($R_1$ = $NH_2$, A = $CHCH_3$, X = S [2-isomer], Y = 3-$CH_3$).

Melting Point: 130-132 °C.

NMR (300 MHz, DMSO-$d_6$): 1.57 (d, 3H); 2.00 (br s, 3H); 6.03 (q, 1H), 7.32-7.44 (m, 2H); 7.71 (s, 1H); 7.80-8.02 (m, 2H); 9.45 (br s, 1H).

Mass Spectrum (CI-$NH_3$): 236 $(M+H)^+$, 253 $(M+NH_4)^+$

Example 11

*N*-hydroxy-*N*-[1-(5-(2,2-dimethylethyl)benzo[b]thien-3-yl)ethyl] urea

a. 4-t-Butyl thiophenol. 4-t-Butyl bromo benzene (10 g, 47 mmole) in ether (100 mL) was heated at reflux for 2 hours in the presence of magnesium metal (1.2 g, 49 mmole). When the Grignard reagent had formed the mixture was cooled to 0°C and sulfur (1.5 g) was added in portions over 10 minutes. The ice bath was removed and the reaction mixture was stirred at room temperature for 1 hour. The mixture was poured into ice water, acidified with 10% HCl and extracted into ether. The organic layer was washed with saturated sodium chloride and dried over $MgSO_4$. The solvent was evaporated to give a cloudy yellow liquid (8.1 g) which was carried on as is.

b. 4-t-Butylphenyl-2-thiapropionaldehyde diethyl acetal. A solution of the thiophenol (prepared as described above, 8.1 g) in THF (25 mL) was added dropwise to a suspension of sodium hydride (2.0 g, 60%, prewashed with hexanes) in THF (50 mL). The mixture was stirred for 30 minutes and then bromo acetaldehyde diethyl acetal (9.3 g. 47 mmole) in ether (25 mL) was added. This mixture was stirred overnight at room temperature. $NH_4Cl$ was added and the solvent was removed *in vacuo*. The residue was partitioned between ether and water. The organic layer was dried over $MgSO_4$ and concentrate. The residue was vacuum distilled to give a yellow oil (5.9 g), bp 142°C/1 mm.

c. 5-t-Butylbenzothiophene. Phosphorous pentoxide and phosphoric acid were combined and heated at 130-135° for 45 minutes. The temperature was raised to 175° and the acetal prepared as in step b above (6.2 g, 2.2 mmole) was added dropwise. The hot solution was dumped onto ice and extracted three times with ether. The combined organic layers were dried over $MgSO_4$ and concentrated to give a brown oil. This oil was filtered through a pad of silica gel, flushed with hexanes. A clear yellow liquid was obtained (2.26 g).

d. *N*-hydroxy-*N*-[1-(5-(2,2-dimethylethyl)benzo[b]thien-2-yl)-ethyl] urea. The desired compound was prepared according to the method of example 2, except using the material prepared as in step c, above, instead of benzothiophene. ($R_1 = NH_2$, $A = CHCH_3$, $X = S$ [2-isomer], $Y = 5\text{-}(CH_3)_3C$).

Melting Point: 151-153°C.

NMR (300 MHz, DMSO-$d_6$): 134 (s, 9H); 1.50 (d, 3H); 5.55 (q, 1H), 6.44 (brs, 2H), 7.22 (s, 1H); 7.37 (dd, 1H); 7.74 (d, 1H); 7.77 (d, 1H); 9.21 (brs, 1H).

Mass Spectrum (CI-$NH_3$): 293 $(M+H)^+$, 310 $(M+NH_4)^+$, 217.

Example 12

*N*-hydroxy-*N*-[1-benzo[b]thien-2-ylethyl) acetamide 1,1-dioxide

a. *N*-acetoxy-*N*-(1-benzo[b]thien-2-ylethyl) acetamide dioxide. Peracetic acid (1.64 g, 8.6 mmole) in acetic acid (5 mL) was added to a solution of *N*-acetoxy-*N*-(1-benzo[b]thien-2-ylethyl acetamide (0.71 g, 2.6 mL), prepared as described in example 1, in acetic acid at 0°C. The mixture was stirred overnight and then poured into saturated aqueous sodium bicarbonate. The aqueous layer was extracted twice with ethyl acetate. The combined organic layers were dried over $MgSO_4$ and concentrated. The residue was chromatographed on 50 g silica gel eluting with 50% ethyl acetate in chloroform. The desired product was obtained as a white foam (0.58 g).

b. *N*-hydroxy-*N*-(1-benzo[b]thien-2-ylethyl) acetamide dioxide was prepared using the procedure of example 1, step e, except using the material prepared as in step a above instead of *N*-acetoxy-*N*-(1-benzo-[b]thien-2-ylethyl) acetamide. ($R_1 = CH_3$, $A = CHCH_3$, $X = SO_2$ [2-isomer], $Y = H$).

Melting Point: 151-157°C.

NMR (300 MHz, DMSO-$d_6$): 1.47 (d, 3H); 2.04 (s, 3H); 5.62 (m, 1H); 7.45 (s, 1H); 7.60 (m, 2H); 7.69 (m, 1H); 7.84 (d, 1H); 9.83 (br s, 1H).

Mass Spectrum (CI-$NH_3$): 268 $(M+H)^+$, 252.

Example 13

*N*-hydroxy-*N*-(1-benzo[b]thien-2-ylethyl) urea 1,1-dioxide

The desired material was prepared using the procedure of example 12, step a, except using *N*-hydroxy-*N*-(1-benzo[b]thien-2-ylethyl) urea instead of N-acetoxy-N-(1-benzo[b]thien-2-ylethyl) acetamide. ($R_1 = NH_2$, $R_2 = CH_3$, $X = SO_2$ [2-isomer], $Y = H$).

NMR (300 MHz, DMSO-$d_6$): 1.42 (d, 3H); 5.29 (m, 2H); 6.57 (brs, 2H); 7.38 (s, 1H); 7.58 (m, 2H); 7.67

(m, 1H); 7.84 (d, 1H); 9.36 (s, 1H).

Example 14

*N*-hydroxy-*N*-(1-benzo[b]fur-2-ylethyl) acetamide

The desired compound was prepared according to the method of example 1, except using benzo[b]-furan instead of benzo[b]thiophene ($R_1 = CH_3$, $A = CHCH_3$, X = O [2-isomer], Y = H)

Melting Point: 130-132 ° C.

NMR (300 MHz, DMSO-$d_6$): 1.44 (d, 3H); 2.36 (s, 3H); 5.77 (q, 1H); 6.37 (br s, 2H); 7.28-7.40 (m, 2H); 7.68-7.73 (m, 1H); 7.88-7.90 (m, 1H); 9.33 (s, 1H).

Mass spectrum (CI-$NH_3$): 222 $(M+H)^+$, 239 $(M+NH_4)^+$

Example 15

*N*-hydroxy-*N*-(1-benzo[b]fur-2-ylethyl) urea

The desired compound was prepared according to the method of example 2, except using benzo[b]-furan instead of benzo[b]thiophene ($R_1 = NH_2$, $A = CHCH_3$, X = O [2-isomer], Y = H)

Melting Point: 147-150 ° C.

NMR (300 MHz, DMSO-$d_6$): 1.46 (d, 3H); 5.47 (q, 1H); 6.48 (brs, 2H); 7.22 (m, 2H); 7.50 (d, 1H); 7.58 (m, 1H); 9.18 (s, 1H).

Mass spectrum (CI-$NH_3$): 221 $(M+H)^+$, 238 $(M+NH_4)^+$, 205, 145.

Example 16

*N*-hydroxy-*N*-(1-(1-methylindol-3-yl)ethyl) acetamide

a. 1-Methyl-2-acetyl indole. 2-Acetyl indole (15 g, 0.94 mmole) and sodium hydroxide (32 g) were dissolved in water (300 mL). Dimethyl sulfate (67.5 g) was added and the mixture was heated at 85 ° for 4 hours. The reaction mixture was then cooled and filtered to collect the product.

b. *N*-hydroxy-*N*-(1-(1-methylindol-3-yl)ethyl) acetamide wasprepared according to the method of example 1, steps b-e, except using the material prepared as in step a above instead of 2-acetyl benzothiophene. ($R_1 = CH_3$, $A = CHCH_3$, $X = NCH_3$ [3-isomer], Y = H).

Melting Point: 106.5-108 ° C.

NMR (300 MHz, DMSO-$d_6$): 1.52 (d, 3H); 1.96 (s, 3H); 5.96 (m, 1H); 7.00 (m, 1H); 7.32 (s, 1H); 7.32 (d, 1H); 7.55 (d, 1H); 9.39 (br s, 1H).

Mass spectrum (EI): 232 $M^+$, 215, 158.

Example 17

*N*-hydroxy-*N*-(1-(1-methylindol-3-yl)ethyl) urea

The desired compound was prepared using the method of example 2, except using 1-methyl-3-acetyl indole, prepared as described in example 16, step a instead of acetyl benzo[b]thiophene. ($R_1 = NH_2$, $A = CHCH_3$, $X = NCH_3$ [3-isomer], Y = H).

Melting Point: 149-150 ° C

NMR (300 MHz, DMSO-$d_6$): 1.46 (d, 3H, J = 7.5 Hz); 3.74 (s, 3H); 5.66 (q, 1H); 6.18 (br s, 2H); 6,94-7.15 (m, 2H); 7.24 (s, 1H); 7.36 (m, 1H); 7.64 (m, 1H); 8.88 (s, 1H).

Mass Spectrum (CI-$NH_3$): 234 $(M+H)^+$, 251 $(M+NH_4)^+$, 158.

Example 18

*N*-hydroxy-*N*-(1-(1-methylindol-3-yl)ethyl) *N'*-methyl urea

The desired compound was prepared using the method of example 2, except using 1-methyl-3-acetyl indole, prepared as described in example 16, step a instead of acetyl benzo[b]thiophene and using methyl isocyanate instead of trimethylsilyl isocyanate. ($R_1 = NHCH_3$, $A = CHCH_3$, $X = NCH_3$ [3-isomer], Y = H).

Melting Point: 152-153 ° C

NMR (300 MHz, DMSO-$d_6$): 1.45 (d, 3H, J = 7.5 Hz); 2.57 (d, 3H); 3.73 (s, 3H); 5.58 (q, 1H); 6.71 (brs, 2H); 6.94-7.15 (m, 2H); 7.24 (s, 1H); 7.35 (m, 1H); 7.62 (m, 1H); 8.74 (s, 1H).

Mass Spectrum (CI-$NH_3$): 248 $(M+H)^+$, 265 $(M+NH_4)^+$, 158.

Example 19

*N*-hydroxy-*N*-(1-benzo[b]thien-2-ylethyl) urea sodium salt

Sodium bistrimethyl silyl amide (10.8 mL, 1.0 M in THF) was added to a solution of *N*-hydroxy-*N*-(1-benzo[b]thien-2-ylethyl) urea (2.5 g, 10.5 mmole), prepared as described in example 2, in THF (50 mL). Hexanes (50 mL) was added and a precipitate formed. The material was collected by filtration and washed with hexanes and ether. After drying *in vacuo* a white solid (1.5 g) was obtained. ($R_1 = NH_2$, A = $CHCH_3$, X = S [2-isomer], Y = H, M = Na).

NMR (300 MHz, DMSO-$d_6$): 1.51 (d, 3H); 2.02 (s, 2H); 4.28, 5.65 (q, 1H); 7.10-7.32 (m, 3H); 7.58-7.75 (m, 2H).

Example 20

*N*-hydroxy-*N*-(1-benzo[b]thien-2-ylethyl) formamide

The desired compound was prepared according to the method of example 1, except using acetyl formyl anhydride instead of acetyl chloride. ($R_1$ = H, A = $CHCH_3$, X = S [2-isomer], Y = H).

Melting Point: 115-116.5 ° C.

NMR (300 MHz, DMSO-$d_6$): 1.61 (s, 3H); 5.4,5.8 (br d, 1H); 7.3-7.4 (m, 3H); 7.77-7.95 (m, 2H); 8.23-8.36 (br d, 1H); 9.8 (br d, 1H).

IR ($CHCl_3$): 160, 2890.

Mass spectrum (CI-$NH_3$): 222 $(M+H)^+$, 239 $(M+NH_4)^+$, 195, 178.

Analysis: Calculated - C 59.71, H 5.01, N 6.33; Found - 59.76; 5.00; 6.31.

Example 21

*N*-hydroxy-*N*-(1-benzo[b]thien-2-ylethyl) 2-methylpropionamide

The desired compound was prepared using the method of example 1, except using isobutyryl chloride instead of acetyl chloride. ($R_1 = (CH_3)_2CH$, A = $CHCH_3$, X = S [2-isomer], Y = H).

Melting Point: 132-138 ° C

NMR (300 MHz, DMSO-$d_6$): 0.98 (d, 3H); 1.06 (d, 3H); 1.57 (d, 3H); 3.04 (m, 1H); 5.90 (q, 1H); 7.28-7.38 (m, 3H); 7.78-7.92 (m, 2H); 9.62 (s, 1H).

IR ($CHCl_3$): 1620, 2980.

Mass spectrum (CI-$NH_3$): 264 $(M+H)^+$, 281 $(M+NH_4)^+$, 195, 178.

Example 22

*N*-hydroxy-*N*-[1-(5-chlorobenzo[b]fur-2-yl)ethyl] urea

a. 2-Acetyl-5-chlorobenzo[b]furan. 5-Chloro salicylaldehyde (15.6 g, 100 mmole) was added to a suspension of potassium carbonate (17.3 g, 125 mmole) in acetone (100 mL). To this was added chloroacetone (90%, 12.0 g) in acetone 50 mL). The mixture was heated at reflux for 24 hours. After cooling, the acetone was removed by evaporation and the residue poured into ice water and 6N HCl. This was extracted with ethyl acetate, which was dried over $Na_2SO_4$ and concentrated *in vacuo* to give the desired material as a crystalline solid (8.24 g).

b. *N*-hydroxy-*N*-[1-(5-chlorobenzo[b]fur-2-yl)ethyl] urea was prepared using the method of example 2, except using the material prepared as in step a, above, instead of 2-acetylbenzothiophene. ($R_1 = NH_2$, A = $CHCH_3$, X = O [2-isomer], Y = 5-Cl).

Melting Point: 173.5- 175 ° C.

NMR (300 MHz, DMSO-$d_6$): 1.46 (d, 3H, J = 7.0); 5.46 (q, 1H, J = 7.0); 6.50 (brs, 2H); 6.73 (s, 1H);. 7.27 (dd, 1H, J = 8.8, J = 2.2); 7.55 (d, 1H, J = 8.8); 7.66 (d, 1H; J = 2.2); 9.21 (s, 1H)

Example 23

*N*-hydroxy-*N*-[1-(5-methoxybenzo[b]fur-2-yl)ethyl] urea

The desired material was prepared using the method of example 22 except using 4-methoxysalicylaldehyde instead of 4-chlorosalicylaldehyde. ($R_1 = NH_2$, $A = CHCH_3$, X = O [2-isomer], Y = 5-$CH_3O$).

Melting point: 149-151 °C.

NMR (300 MHz, DMSO-$d_6$): 1.44 (d, 3H, J = 7.0); 3.76 (s, 3H); 5.43 (q, 1H, J = 7.0); 6.47 (brs, 2H); 6.65 (m, 1H); 6.83 (dd, 1H, J = 2.6, J = 9.2); 7.08 (d, 1H, J = 2.6); 7.39 (d, 1H, J = 92); 9.17 (s, 1H).

Example 24

*N*-hydroxy-*N*-(1-(1-methylindol-2-yl)ethyl) urea

The desired compound was prepared using the method of example 2, except using 1-methyl-2-acetyl indole, prepared as described in example 16, step a instead of acetyl benzo[b]thiophene. ($R_1 = NH_2$, $A = CHCH_3$, X = $NCH_3$ [2-isomer], Y = H).

Melting Point: 137-139 °C

NMR (300 MHz, DMSO-$d_6$): 1.48 (d, 3H, J = 7 Hz); 3.75 (s, 3H); 5.70 (q, 1H); 6.15 (br s, 2H); 6..70 (s, 1H); 6.94 (m, 1H); 7.15 (m, 1H); 7.35 (m, 1H); 7.65 (m, 1H); 8.90 (s, 1H).

IR: 3300, 1660, 1580.

Mass Spectrum (CI-$NH_3$): 234 $(M + H)^+$, 251 $(M + NH_4)^+$, 158.

Example 25

*N*-hydroxy-*N*-(1-benzo[b]thien-2-ylethyl) thiourea

The desired compound was prepared according to the method of example 2, method a, except using trimethylsilyl isothiocyanate instead of trimethylsilyl isocyanate ($R_1 = NH_2$, $A = CHCH_3$, X = S [2-isomer], Y = H, Z = S).

Melting Point: 165 °C dec.

NMR (300 MHz, DMSO-$d_6$): 1.58 (3, d, J = 7.5 Hz); 6.83 (1, m); 7.26-7.37 (3, m); 7.53-7.62 (1, br s); 7.75-7.92 (2, m); 7.92-8.02 (1, br s); 9.28 (1, br s).

Mass spectrum (DCI-$NH_3$): 253 $(M + H)^+$, 270 $(M + NH_4)^+$, 237, 161.

Analysis ($C_{11}H_{12}N_2OS_2$): Calculated -- C: 52.36, H: 4.79, N: 11.10; Found C: 52.60, H: 4.88, N: 11.04.

Example 26

*N*-hydroxy-*N*-[1-(3-methoxybenzo[b]thien-2-yl)ethyl] urea

a. 2-(2-Oxothiopropoxy)benzoic acid. Potassium carbonate (8.97 g, 64.9 mmole) was added to a solution of thiosalicylic acid (10 g, 65 mmole) in ethanol (70 mL). The mixture was heated at reflux for 3 hours and then the solvent was evaporated. The residue was dissolved in water and the solution acidified to pH 1 with concentrated HCl. The desired product precipitated and was collected and recrystallized from ethyl acetate / hexanes. Two crops were collected for a total of 10.2 g (81%) of the desired product.

b. Methyl 2-(2-oxothiopropoxy)benzoic acid. The material prepared as described in step a, above (2.0 g, 10.3 mmole) was suspended in ether and excess diazomethane in ether was added. The residual diazomethane was destroyed by the addition of aqueous HCl. The ether layer was dried over $MgSO_4$ and the solvent removed *in vacuo* to give the desired product (1.95 g, 85%).

c. 3-Hydroxy-2-acetylbenzo[b]thiophene. Sodium methoxide (230 mg, 4.3 mmole) was added to a solution of the ester prepared as in step b, above (1.9 g, 8.5 mmole) in methanol (40 mL). Chloroacetone (6.2 mL, 77 mmole) was added and the reaction mixture was heated at reflux for four hours and then allowed to stir for fifteen hours at room temperature. The solvent was removed *in vacuo* and the residue partitioned between 2N NaOH and ethyl acetate. The aqueous phase was acidified with concentrated HCl and the desired product precipitated. It was collected by filtration (1.9 g, 85%).

d. 3-Methoxy-2-acetylbenzo[b]thiophene. The material prepared as in step c was converted to the desired product by treatment with diazomethane using the procedure described in step b, above.

e. N-hydroxy-N-[1-(3-methoxybenzo[b]thien-2-yl)ethyl] urea was prepared according to the method of example 2, method a, except using the material prepared as in step d, above instead of 2-acetylbenzo[b]-thiophene. ($R_1 = NH_2$, A = $CHCH_3$, X = S [2-isomer], Y = 3-$OCH_3$).

Melting Point: 158 ° C dec.

NMR (300 MHz, DMSO-$d_6$): 1.42 (d, 3H, 1 = 7.5 Hz); 3.91 (s, 3H); 5.81 (q, 1H, J = 7.5 Hz); 6.42 (br s, 2H); 7.30-7.41 (m, 2H); 7.66-7.73 (m, 1H); 7.82-7.88 (m, 1H); 9.44 (s, 1H).

Mass spectrum (DCI-$NH_3$): 267 $(M+H)^+$, 284 $(M+NH_4)^+$.

Analysis ($C_{12}H_{14}N_2O_3S$): Calculated -- C: 54.12, H: 5.30, N: 10.52; Found C: 54.11, H: 5.34, N: 10.27.

Example 27

N-hydroxy-N-[1-(3-thioethylbenzo[b]thien-2-yl)ethyl] urea

a. 1-(3-thioethoxybenzo[b]thien-2-yl)ethyl hydroxylamine. A solution of 1-(3-methoxybenzo[b]thien-2-yl)-ethyl hydroxylamine (250 mg, 1.1 mmole), prepared as described in example 26 in methylene chloride (5 mL), was added to a solution of aluminum chloride (895 mg, 6.7 mmole) in ethanethiol (4 mL) at 0 ° C. The cold bath was removed and the mixture was allowed to stir for 3 hours. The mixture was poured onto ice and extracted into ethyl acetate. The organic layer was dried over $MgSO_4$ and evaportated to give the desired product as a yellow oil (250 mg).

b. N-hydroxy-N-[1-(3-thioethylbenzo[b]thien-2-yl)ethyl] urea The desired product was obtained using the method of example 2, method a, except using the material prepared as in step a, above, instead of 1-(benzo[b]thien-2-yl)ethyl hydroxylamine ($R_1 = NH_2$, A = $CHCH_3$, X = S [2-isomer], Y = 3-$SC_2H_5$).

Melting Point: 153-155 ° C

NMR (300 MHz, DMSO-$d_6$): 1.10 (t, 3H, J = 7.5 Hz); 1.44 (d, 3H, J = 7.0 Hz); 2.70 (m, 2H); 6.18 (q, 1H, J = 7.0 Hz); 6.45 (br s, 2H); 7.41 (m, 2H); 7.93 (m, 2H); 9.57 (s, 1H).

Mass spectrum (DCI-$NH_3$): 297 $(M+H)^+$, 314 $(M+NH_4)^+$, 221.

Analysis ($C_{13}H_{16}N_2O_2S_2$): Calculated -- C: 52.68, H: 5.44, N: 9.45; Found C: 52.35, H: 5.41, N: 9.26.

Example 28

N-hydroxy-N-[1-(4,7-dimethylbenzo[b]thien-2-yl)ethyl] urea

The desired compound was prepared according to the method of example 11 except using 2,5-dimethyl bromobenzene instead of 4-t-butylbromobenzene ($R_1 = NH_2$, A = $CHCH_3$, X = S [2-isomer], Y = 4,7-$(CH_3)_2$).

Melting Point: 149-152 ° C

NMR (300 MHz, DMSO-$d_6$): 1.52 (d, 3H, J = 7.5 Hz); 2.42 (s, 3H); 2.50 (s, 3H); 5.60 (m, 1H); 6.46 (b s, 2H); 7.04 (m, 2H); 7.32 (m, 1H); 9.26 (s, 1H).

Mass spectrum (DCI-$NH_3$): 265 $(M+H)^+$, 282 $(M+NH_4)^+$, 189.

Example 29

N-hydroxy-N-[1-(5-fluorobenzo[b]thien-2-yl)ethyl] urea

a. 5-Fluorophenylbenzo[b]thiophene was prepared using the method of example 11, steps b, c, except using 4-fluorothiophenol instead of 4-t-butyl thiophenol.

b. N-benzyloxy-1-(5-fluorobenzo[b]thien-2-yl)ethyl amine. n-Butyl lithium (2.5M in hexanes, 4.4 mL) was added to a solution of 5-fluorobenzothiophene, prepared as described in part a, above (1.52 g, 10 mmole) in THF (25 mL) at -78 ° C. After stirring for 30 minutes borontrifluoride etherate was added followed by *O*-benzyl acetaldehyde oxime. Fifteen minutes later aqueous $NH_4Cl$ was added and the reaction warmed to room temperature. The aqueous phase was extracted twice with ether and the combined organic layers washed twice with brine, dried over $MgSO_4$ and concentrated The desired product was obtained as a yellow oil (2.9 g).

c. 1-(5-Fluorobenzo[b]thien-2-yl)ethyl hydroxylamine. The material prepared as in step a, above (1.48 g, 4.91 mmole) in methylene chloride (2 mL) was added to a solution of aluminum chloride in ethanethiol (15 mL) at 0 ° C. The ice bath was removed and the mixture was allowed to stir for 5 hours at room temperature. The mixture was poured onto ice and acidified with 10% aqueous HCl. The aqueous layer was extracted with ethyl acetate and the combined organic phases was dried over $MgSO_4$ and concentrated to give the desired product as a pale yellow powder (0.83 g).

d. N-hydroxy-N-[1-(5-fluorobenzo[b]thien-2-yl)ethyl] urea was prepared using the method of example 2, method a, except using the material prepared as in step c, above, instead of 1-(2-benzo[b]thienyl) ethyl hydroxyl amine. ($R_1 = NH_2$, A = $CHCH_3$, X = S [2-isomer], Y = 5-F).

Melting Point: 149-153 °C.

NMR (300 MHz, DMSO-$d_6$): 1.50 (d, 3H, J = 7.5 Hz); 5.56 (m, 1H); 6.47 (br s, 2H); 7.13-7.29 (m, 2H); 7.57-7.77 (m, 1H); 7.81-7.95 (m, 1H); 9.26 (m, 1H).

Mass spectrum (DCI-$NH_3$): 255 $(M+1)^+$, 272 $(M+NH_4)^+$.

Analysis ($C_{11}H_{11}FN_2O_2S$): Calculated -- C: 51.96, H: 4.36, N: 11.02; Found C: 52.05, H: 4.63, N: 10.46.

Example 30

N-hydroxy-N-(2-benzo[b]thien-2-yl-1-methylethyl) urea

a. 3-Benzo[b]thien-2-yl-2-nitroprop-2-ene. Sodium hydroxide (4 M solution, 3.2 mL) was added to a solution of benzo[b]thiophene-2-carboxaldehyde (2.0 g, 12.3 mmole) and nitroethane (0.9 mL, 12.3 mmole) in methanol. The reaction was stirred at 5 °C for 1 hour and then allowed to warm to room temperature and stirred for 15 hours. The mixture was added to 3 ml of 6 N HCl and the desired product precipitated. It was collected by filtration and chromatographed on silica gel, eluting with 20% ether in hexanes.

b. 2-Benzo[b]thien-2-yl-1-methylethyl hydroxyl amine. A solution of the material prepared as in part a, above (0.56 g, 1.6 mmole) in THF (4 mL) was added to borane-THF complex at 0 °C. The ice bath was removed and sodium borohydride (5 mg) was added. After being allowed to be stirred for 90 minutes ice water (~10 mL) was added followed by 10% HCl (3 mL). The solution was extracted with ether, then the aqueous layer was made basic by the addition of 2 N NaOH and extracted with ether. This ether phase was dried over $MgSO_4$ and evaporated to give the desired product (70 mg).

c. N-hydroxy-N-(2-benzo[b]thien-2-yl-1-methylethyl) urea. The desired material was prepared using the method of example 2, method a, except using the the material prepared as in step b, above, instead of 1-benzo[b]thien-2-ylethyl hydroxyl amine. ($R_1 = NH_2$, A = $CH_2CHCH_3$, X = S [2-isomer], Y = H).

Melting Point: 141-143 °C.

NMR (300 MHz, DMSO-$d_6$): 1.05 (3H, d, d J = 7.5 Hz); 2.88-3.11 (2H, m); 4.43 (1H, m); 6.33 (2H, br s); 7.18-7.36 (3H, m); 7.70-7.90 (2H, m); 9.07 (1H, br s).

Mass spectrum (DCI-$NH_3$): 251 $(M+H)^+$, 268 $(M+NH_4)^+$, 235, 208, 192.

Analysis ($C_{12}H_{14}N_2O_2S$): Calculated -- C: 57.58, H: 5.64, N: 11.19; Found C: 57.68, H: 5.67, N: 11.14.

Example 31

N-hydroxy-N-(3-benzo[b]thien-2-ylprop-2-enyl) acetamide

a. 3-Benzo[b]thien-2-ylbromoprop-2-ene. Vinyl magnesium bromide (14.8 mmole) was added to a solution of 2-benzo[b]thiophene carboxaldehyde (2.0 g, 12.3 mmole) in THF (50 mL) at 0 °C. After fifteen minutes the reaction mixture was cooled to -25 °C and HBr (48%, 12 mL) was added dropwise so that the temperature remained below 0 °C. The mixture was allowed to warm to room temperature and allowed to stir for 1 hour. The solvent was then removed *in vacuo*. The residue was partitioned between water and ethyl acetate. The organic phase was evaporated to give a brown solid which was recrystallized from hexane / ethyl acetate. The desired product was obtained as a yellow solid (800 mg).

b. O-Tetrahydropyranyl-3-benzo[b]thien-2-ylprop-2-enyl hydorxylamine. Tetrahydropyranylhydroxyl amine (0.51 g, 4.34 mmole) in DMF (10 mL) was added to a solution of 3-benzo[b]thienylbromoprop-2-ene (0.50 g, 1.98 mmole) in DMF (10 mL). After being allowed to stir for two hours the reaction was poured into water and extracted with ether. The organic layer was washed with $NaHCO_3$ and brine. The organic layer was dried over $MgSO_4$ and evaporated. The residue was chromatographed on silica gel eluting with 40% ether in hexanes to give the desired product as a yellow oil (66%).

c. 3-Benzo[b]thien-2-ylprop-2-enyl hydroxylamine. A few drops of concentrated HCL was added to a solution of the material prepared as in part b, above (5.4 g) in methanol (150 mL) and stirred for three days. The solvent was removed to afford the desired product as its hydrochloride salt.

d. N-hydroxy-N-(3-benzo[b]thien-2-ylprop-2-enyl) acetamide was prepared using the method of example 1, steps d and e, except using the material prepared as in step c above, instead of 1-benzo[b]thien-2-ylethyl hydroxylamine. ($R_1 = CH_3$, A = CH = $CHCH_2$, X = S [2-isomer], Y = H).

Melting Point: 168 °C dec.

NMR (300 MHz, DMSO-$d_6$): 204 (s, 3H); 4.30 (m, 2H); 6.06 (m, 1H); 6.88 (m, 1H); 7.35 (m, 3H); 7.83 (m,

2H); 10.05 (br s, 1H).

Mass spectrum (DCI-$NH_3$): 248 $(M+H)^+$, 265 $(M+NH_4)^+$, 232, 173.

Analysis ($C_{13}H_{13}NO_2S$): Calculated -- C: 63.14, H: 5.30, N: 5.66; Found C: 61.13, H: 5.22, N: 5.43.

Example 32

*N*-hydroxy-*N*-(3-benzo[b]thien-2-ylprop-2-enyl) urea

The desired material was prepared according to the method of example 2, method a, except using 3-benzo[b]thienylprop-2-enyl hydroxylamine, prepared as in example 31, step c instead of 1-benzo[b]thien-2-ylethyl hydroxylamine. ($R_1 = NH_2$, A = $CH=CHCH_2$, X = S [2-isomer], Y = H).

Melting Point: 167 °C dec.

NMR (300 MHz, DMSO-$d_6$): 4.13 (m, 2H); 6.10 (m, 1H); 6.43 (br s, 2H); 6.87 (m, 1H); 7.34. (m, 3H); 7.83 (m, 2H); 9.44 (s, 1H).

Mass spectrum (DCI-$NH_3$): 249 $(M+1)^+$, 266 $(M+NH_4)^+$, 233, 173.

Analysis ($C_{12}H_{12}N_2O_2S$): Calculated -- C: 58.05, H: 4.87, N: 11.28; Found C: 57.74, H: 4.97, N: 11.00.

Example 33

*N*-hydroxy-*N*-[1-(5,7-dichlorobenzo[b]fur-2-yl)ethyl] urea

The desired material was prepared according to the method of example 22, except using 3,5-dichlorosalicylaldehyde, instead of 5-chlorosalicylaldehyde. ($R_1 = NH_2$, A = $CHCH_3$, X = O [2-isomer], Y = 5,7-$Cl_2$).

Melting Point: 140-142 °C

NMR (300 MHz, DMSO-$d_6$): 1.46 (d, 3, J = 6.8 Hz); 5.48 (q, 1, J = 6.8 Hz); 6.56 (br s, 2); 6.85 (d, 1, J = 0.7 Hz); 7.51 (d, 1, J = 1.8 Hz); 7.69 (d, 1, J = 1.8 Hz); 9.23 (s, 1).

Mass spectrum (CI-$NH_3$): 289 $(M+1)^+$, 306 $(M+NH_4)^+$.

Analysis ($C_{11}H_{10}Cl_2N_2O_3$): Calculated -- C: 45.69, H: 3.49, N: 9.69; Found C: 45.78, H: 3.62, N: 9.57.

Example 34

*N*-hydroxy-*N*-[1-(7-methoxybenzo[b]fur-2-yl)ethyl urea

The desired material was prepared according to the method of example 22, except using 3-methoxysalicylaldehyde, instead of 5-chlorosalicylaldehyde. ($R_1 = NH_2$, A = $CHCH_3$, X = O [2-isomer], Y = 7-$CH_3O$).

Melting Point: 136-137.5 °C.

NMR (300 MHz, DMSO-$d_6$): 1.44 (d, 3, J-7.0 Hz); 3.90 (s, 3); 5.44 (q, 1, J = 7.0 Hz); 6.46 (br s, 2); 6.69 (d, 1, J = 1.1); 6.86 (m, 1); 7.11 (m, 2); 9.15 (s, 1).

Mass spectrum (CI-$NH_3$): 251 $(M+1)^+$, 268 $(M+NH_4)^+$, 175.

Analysis ($C_{12}H_{14}N_2O_4$): Calculated -- C: 57.59, H: 5.64, N: 11.20; Found C: 57.71, H: 5.70, N: 11.21.

Example 35

*N*-hydroxy-*N*-[1-(7-methoxybenzo[b]fur-2-yl)ethyl] *N'*-methyl urea

The desired material was prepared according to the method of example 22, except using 3-methoxysalicylaldehyde, instead of 5-chlorosalicylaldehyde and using methyl isocyanate instead of trimethylsilyl isocyanate ($R_1 = NHCH_3$, A = $CHCH_3$, X = O [2-isomer], Y = 7-$CH_3O$).

Melting Point: 135-137 °C.

NMR (300 MHz, DMSO-$d_6$): 1.44 (d, 3, J = 7.0 Hz); 2.63 (d, 3, J = 4.4 Hz); 3.91 (s, 3); 5.43 (q, 1, J = 7.0 Hz); 6.69 (d, 1, J = 1.1 Hz); 6.86 (dd, 1, J = 2.7 Hz, J = 6.1 Hz); 6.99 (br q, 1, J = 4.4); 7.12 (m, 2); 9.09 (s, 1).

Mass spectrum (CI-$NH_3$): 265 $(M+1)^+$, 282 $(M+NH_4)^+$.

Analysis ($C_{13}H_{16}N_2O_4$): Calculated -- C: 59.08, H: 6.10, N: 10.60; Found C: 59.16, H: 6.09, N: 10.60.

Example 36

*N*-hydroxy-*N*-[1-(7-methoxybenzo[b]fur-2-yl)ethyl] urea

The desired material was prepared according to the method of example 22, except using 3-ethoxysalicylaldehyde, instead of 5-chlorosalicylaldehyde. ($R_1 = NH_2$, A = $CHCH_3$, X = O [2-isomer], Y = 7-$CH_3CH_2O$).

Melting Point: 146-148 °C.

NMR (300 MHz, DMSO-$d_6$): 1.40 (t, 3, J = 7.0); 1.44 (d, 3, J = 7.0); 4.20 (q, 2, J = 7.0); 5.45 (q, 1, J = 7.0); 6.49 (br s, 2); 6.70 (d, 1, J = 0.7); 6.85 (dd, 1, J = 2.2, J = 6.6); 7.11 (m, 2); 9.14 (s, 1).

Mass spectrum (CI-$NH_3$): 265 $(M + 1)^+$, 282 $(M + NH_4)^+$, 189.

Analysis ($C_{13}H_{16}N_2O_4$): Calculated -- C: 59.08, H: 6.10, N: 10.60; Found C: 59.06, H: 6.17, N: 10.50.

Example 37

*N*-hydroxy-*N*-(1-indol-2-ylethyl] *N'*-methyl urea

a. 2-Acetylindole. n-Buryl lithium (40 mL, 2.5 M in hexanes, 100 mmole) was added dropwise to a solution of indole (11.7 g, 100 mmole) in dry THF (100 mL) at -70 °C. After thirty minutes the mixture was added via cannula to an excess of carbon dioxide (dry ice, 18 g) in THF (100 mL). The solution was then allowed to warm to room temperature. The solvent was evaporated ***in vacuo*** to give indole-1-carboxylic acid, lithium salt, as a white solid. Benzene (50 mL) was added to the mixture and distilled away to remove traces of water. Fresh THF (100 mL) was added to the solid and the solution was cooled to -70 °C. t-Butyl lithium (63 mL, 1.6 M in pentane, 100 mmole) was slowly added to the solution. The mixture was allowed to stir at -70 °C for 1 hour and then ***N,O***-dimethyl acetohydroxamic acid (10.3 g, 100 mmole) was added in THF (100 mL). The solution was stirred for an additional 2 hours at -70 °C and then at 0 °C for 1 hour. The reaction was quenched by the addition of ammonium chloride (20 mL, saturated). The mixture was extracted with ethyl acetate, and the organic phase dried over sodium sulfate and the solvent removed ***in vacuo***. The residue was chromatographed on silica gel, eluting with methylene chloride to afford the desired material as a white solid (10.4 g, 66%).

b. *N*-hydroxy-*N*-(1-indol-2-ylethyl) *N'*-methyl urea. The desired product was obtained using the method of example 2, method a, except using the material prepared as in step a, above instead of 2-acerylbenzothiophene and using methyl isocyanate instead of trimethylsilyl isocyanate. ($R_1 = NHCH_3$, A = $CHCH_3$, X = NH [2-isomer], Y = H).

Melting Point: 164-165 °C.

NMR (300 MHz, DMSO-$d_6$): 1.48 (d, 2H, J = 7 Hz); 3.60 (br s, 3H); 5.51 (q, 1H, J = 7 Hz); 6.31 (br s, 1H); 6.95 (m, 1H); 7.05 (m, 1H); 7.32 (m, 1H); 7.45 (m, 1H); 9.21 (s, 1H); 10.82 (br s, 1H).

IR (KBr): 3310, 2980, 2920, 1660, 1580, 1480, 1460, 1420, 1375, 1320, 1220, 1240, 1140, 1120, 1000, 900, 860, 800, 760, 740, 680, 600, 495.

Mass spectrum (DCI-$NH_3$): 234 $(M + H)^+$, 247 $(M + NH_4)^+$, 144.

Example 38

*N*-hydroxy-*N*-[1-(5-chloroindol-2-yl]ethyl] *N'*-methyl urea

The desired material was prepared according to the method of example 37, except using 5-chloroindole, instead of indole and using trimethylsilyl isocyanate instead of methylisocyanate ($R_1 = NH_2$, A = $CHCH_3$, X = NH [2-isomer], Y = 5-Cl).

Melting Point: 159-160 °C.

NMR (300 MHz, DMSO-$d_6$): 1.55 (d, J = 7 Hz, 3H); 5.52 (q, J = 7 Hz, 1H); 6.35 (s, 1H); 6.51 (br s, 2H); 7.10 (m, 1H); 7.43 (m, 1H); 7.58 (m, 1H); 9.21 (s, 1H); 11.15 (s, 1H).

IR: (KBr): 3460, 3420, 3350, 3280, 3200, 3000, 1660, 1580, 1460, 1440, 1420, 1320, 1160, 1120, 1060, 1000, 900, 880, 860, 800, 760, 720, 690, 660, 600, 470.

Mass spectrum (DCI-$NH_3$): 254 $(M + H)^+$, 271 $(M + NH_4)^+$, 178.

Example 39

N-hydroxy-N-[1-(1-acetylindol-2-yl)ethyl] urea

a. N-hydroxy-N-(1-indol-2-yl)ethyl urea was prepared according to the method of example 37, except using trimethylsilyl isocyanate instead of methylisocyanate. (See also example 53.)

b. N-acetoxy-N-[1-(1-acetylindol-2-yl)ethyl] urea. Lithiumcyanide (4 mL of a 0.5 M solution in DMF) was added to a solution of the material prepared as in step a, above (0.22 g, 1 mmole) in THF (20 mL). Acetyl chloride (0.14 mL, 2 mmole) in THF (5 mL) was then added dropwise. After 3 hours the solvent was removed *in vacu*o and the residue partitioned between water (30 mL) and ethyl acetate (60 mL). The organic layer was separated and washed with brine and dried over sodium sulfate. The solvent was evaporated and the residue recrystallized from ether / ethyl acetate to give the desire product as a colorless solid (58%, 175 mg).

c. N-hydroxy-N-[1-(1-acetylindol-2-yl)ethyl] urea. The material prepared as in step b, above (175 mg) was dissolved in isopropanol / water (2/1, 20 mL) and lithium hydroxide (50 mg, 1.2 mmole) was added in water(1 mL). The mixture was allowed to stir for 30 min, saturated ammonium chloride was added (50 mL) and the desired product extracted into ethyl acetate. The organic phase was washed with brine and dried over sodium sulfate. The solvent was removed *in vacuo* and the residue recrystallized from ether / ethyl acetate to afford the desired product as a white solid (135 mg, 73%). ($R_1 = NH_2$, $A = CHCH_3$, $X = NCOCH_3$ - [2-isomer], $Y = H$).

Melting Point: 146-147 ° C.

NMR (300 MHz, DMSO-$d_6$): 1.52 (d, 3H, J = 7 Hz); 2.21 (s, 3H); 5.45 (q, 1H, J = 7 Hz); 6.32 (s, 1H); 6.55 (br s, 2H); 6.95 (m, 1H); 7.05 (m, 1H); 7.35 (m, 1H); 7.45 (m, 1H); 10.52 (s, 1H).

IR (KBr) : 3300, 3150; 3000, 2900, 1660, 1650, 1500, 1380, 1220, 1150, 1100, 1000, 950, 780, 750.

Mass spectrum (DCI-$NH_3$): 262 $(M+H)^+$, 279 $(M+NH_4)^+$, 219.

Example 40

N-hydroxy-N-[1-(1-methanesulfonylindol-2-yl)ethyl] urea

The desired material was prepared as described in example 37, except using 1-methanesulfonyl indole instead of indole and using trimethylsilyl isocyanate instead of methyl isocyanate. ($R_1 = NH_2$, $A = CHCH_3$, $X = NSO_2CH_3$ [2-isomer], $Y = H$).

Melting Point: 166-167 ° C.

NMR (300 MHz, DMSO-$d_6$): 1.50 (d, 3H, J = 7 Hz); 3.55 (s, 3H); 5.42 (q, 1H, J = 7 Hz); 6.35 (s, 1H); 6.50 (br s, 2H); 6.95. (m, 1H); 7.08 (m, 1H); 7.32 (m, 1H); 7.43 (m, 1H); 10.47 (s, 1H).

IR (KBr): 3300, 3150, 2900, 1650, 1340, 1160, 1495, 1215, 1100.

Mass spectrum (DCI-$NH_3$): 298 $(M+H)^+$, 315 $(M+NH_4)^+$.

Example 41

N-hydroxy-N-benzo[b]thien-7-ylmethyl urea

a 7-Methylbenzo[b]thiophene was prepared using the method of example 11, steps b, c, except using 2-methylthiophenol instead of 4-t-butyl thiophenol.

b. 7-Bromomethylbenzo[b]thiophene. N-bromosuccinimide (4.32 g, 24.3 mmole) and the material prepared as in step a, above (3.61 g, 24.3 mmole) were suspended in $CCl_4$ (50 mL). Benzoyl peroxide (0.59 g, 2.43 mmole) was added and the suspension was heated at reflux for 3 hours. The reaction mixture was cooled to room temperature and solid filtered off. The filtrate was dried over $MgSO_4$ and concentrated *in vacuo*. The residue was chromatographed on silica gel eluting with hexane followed by ether to afford the desired product.

c. 7-Hydroxymethylbenzo[b]thiophene. The material prepared as in step b, above (1.3 g, 5.7 mmole) was dissolved in dioxane (15 mL) and 2N NaOH was added. The mixture was allowed to stir three days at room temperature. The mixture was extracted twice with ether and the combined organic layers were washed with brine, dried over $MgSO_4$ and concentrated. The residue was chromatographed on silica gel eluting with 50% ether in hexanes to afford the desired product as a yellow powder (0.49 g).

d. Benzo[b]thiophene-7-carboxaldehyde. A solution of the material prepared as in step c, above (0.49 g) in benzene was added to a suspension of manganese dioxide on carbon (5.0 g) and heated at reflux for 3

hours. The reaction mixture was filtered and concentrated to afford the desired product as a yellow oil (0.4 g).

e. *N*-hydroxy-*N*-benzo[b]thien-7-ylmethyl urea was prepared using the method of example 2, method a, except using the material prepared as in step d, above, instead of 2-acetylbenzo[b]thiophene. ($R_1 = NH_2$, $A = CH_2$, X = S [7-isomer], Y = H).

Melting Point: 138-140.5 °C.

NMR (300 MHz DMSO-$d_6$): 4.77 (s, 2H); 6.46 (br s, 2H); 7.30 (m, 1H); 7.37 (m, 1H); 7.49 (d, 1H, J = 6 Hz); 7.75 (d, 1H, 6 Hz); 7.81 (m, 1H); 9.39 (s, 1H).

Mass spectrum (DCI-$NH_3$): 223 $(M+H)^+$, 240 $(M+NH_4)^+$.

Analysis ($C_{10}H_{10}N_2O_2S$): Calculated -- C: 54.04, H: 4.54, N: 12.60; Found C: 53.87, H: 4.67, N: 12.44.

Examples 42

*N*-hydroxy-*N*-[1-(2,3-dihydrobenzo[b]fur-yl)ethyl] urea

a. 1-(2,3-dihydrobenzo[b]fur-2-yl)ethanol. A suspension of palladium on carbon (10%, 220 mg) and 2-acetyl benzo[b]furan (19.5 g, 122 mmole) was allowed to stir for 48 hours under an atmosphere of hydrogen (pressure = 1 atm). The mixture was filtered through celite and the filtrate was concentrated. The residue was chromatographed on silica gel (300 g) eluting with 25% ethyl acetate in hexanes to afford the desired product.

b. 2-Acetyl-2,3-dihydrobenzo[b]furan. A solution of dry DMSO (18.0 g, 230 mmole) in methylene chloride (30 mL) was added to a solution of oxalyl chloride (14.5 g, 114.2 mmole) in methylene chloride (100 mL) at -60 °C. Thirty minutes later the alcohol prepared as in step a, above (15.1 g, 92 mmole) in methylene chloride (75 mL) was added. The resulting suspension was stirred at -60 °C. for 1 hour and then triethylamine (48 g, 474 mmole) was added. The mixture was allowed to stir at room temperature for 15 hours. The mixture was washed with water, 5% HCl, saturated NaCl, and dried over $MgSO_4$. The solvent was removed *in vacuo* to yield the desired product as a light yellow oil.

c. *N*-hydroxy-*N*-[1-(2,3-dihydrobenzo[b]fur-2-yl)ethyl] urea. The desired material was prepared using the method of example 2, method a, except using the material prepared as described in step b, instead of 2-acetylbenzo[b]thiophene. ($R_1 = NH_2$, $A = CHCH_3$, X = O [2-isomer], Y = 2,3-dihydro)

Melting Point: 129-131 °C.

NMR (300 MHz, DMSO-$d_6$): Mixture of diasteriomers 1.04, 1.17 (d, 3 J = 6.8); 2.98 (m, 1); 3.22 (m, 1); 4.07, 4.34 (m, 1); 4.66, 4.75 (m, 1); 6.29, 6.41 (br s, 2); 6.67-6.80 (m, 2); 7.07, 7.19 (m, 1); 9.12, 9.15 (s, 1).

Mass spectrum (DCI-$NH_3$): 223 $(M+H)^+$, 240 $(M+NH_4)^+$.

Analysis ($C_{11}H_{14}N_2O_3$): Calculated -- C: 59.44, H: 6.35, N: 12.61; Found C: 59.41, H: 6.37, N: 12.60.

Example 43

*N*,*N*'-dihydroxy-*N*-(1-benzo[b]thien-2-ylethyl) urea

The desired material was prepared according to the method of example 2, method b, except using hydroxylamine instead of ammonium hydroxide. ($R_1 = NHOH$, $A = CHCH_3$, X = S [2-isomer], Y = H).

Melting Point: 132-134 °C dec.

NMR (300 MHz, DMSO-$d_6$): 1.52 (3H, d, J = 7.5 Hz); 5.49 (1H, q, J = 7.5 Hz); 7.26-7.37 (3H, m); 7.74-7.92 (2H, m); 8.42 (1H, br s); 9.15 (1H, br s); 9.00 (1H, br s).

Mass spectrum (DCI-$NH_3$): 253 $(M+H)^+$, 270 $(M+NH_4)^+$, 237, 221, 176, 161.

Example 44

*N*-hydroxy-*N*-(1-benzo[b]thien-2-ylethyl) *N*'-ethylurea

The desired material was prepared according to the method of example 2, method a, except using ethyl isocyanate instead of trimethylsilyl isocyanate. ($R_1 = NHCH_2CH_3$, $A = CHCH_3$, X = S [2-isomer], Y = H).

Melting Point: 138-139 °C.

NMR (300 MHz, DMSO-$d_6$): 1.01 (t, 3H, J = 7.5 Hz); 1.52 (d, 3H, J = 7.5 Hz); 3.00-3.18 (m, 2H); 5.56 (q, 1H; J = 7.5); 7.05 (m, 1H); 7.22-7.40 (m, 3H); 7.70-7.95 (m, 2H); 9.18 (g, 1H).

Mass spectrum (DCI-$NH_3$): 265 $(M+H)^+$, 282 $(M+NH_4)^+$, 176, 161, 157.

Analysis ($C_{13}H_{16}N_2O_2S$): Calculated -- C: 59.07, H: 6.10, N: 10.60; Found C: 58.92, H: 6.24, N: 10.58.

Example 45

*N*-hydroxy-*N*-(1-benzo[b]thien-2-ylethyl) *N'*-methyl thiourea

The desired material was prepared according to the method of example 2, method a, except using methyl isothiocyanate instead of trimethylsilyl isocyanate. ($R_1$ = $NHCH_3$, A = $CHCH_3$, X = S [2-isomer], Y = H, Z = S).

Melting Point: 154-155 °C.

NMR (300 MHz, DMSO-$d_6$): 1.59 (d, 3H, J = 7.5 Hz); 2.91 (d, 3H, J = 4.5 Hz); 6.74 (qt, 1H, J = 7.5 Hz); 7.25-7.40 (m, 3H); 7.75-7.93 (m, 2H); 8.38 (q, 1H, J = 4.5 Hz); 9.68 (s, 1H).

Mass spectrum (DCI-$NH_3$): 267 $(M+H)^+$, 284 $(M+NH_4)^+$, 194, 176, 161.

Analysis ($C_{12}H_{14}N_2OS_2$): Calculated -- C: 54.11, H: 5.30, N: 10.52; Found C: 53.98, H: 5.31, N: 10.49.

Example 46

*N*-hydroxy-*N*-benzo[b]thien-2-ylmethyl *N'*-methyl urea

The desired material was prepared according to the method of example 2, method a, except using 2-benzo[b]thiophene carboxaldehyde instead of 2-acetylbenzo[b]thiophene and using methyl isocyanate instead of trimethylsilyl isocyanate. ($R_1$ = $NHCH_3$, A = $CH_2$, X = S [2-isomer], Y = H).

Melting Point: 160-161 °C.

NMR (300 MHz, DMSO-$d_6$): 2.61 (d, 3H, J = 4.5 Hz); 4.74 (s, 2H); 7.01 (q, 1H, J = 4.5 Hz); 7.26-7.38 (m, 3H); 7.74-7.97 (m, 2H); 9.46 (s, 1H)

Mass spectrum (CI-$NH_3$): 237 $(M+1)^+$, 254 $(M+NH_4)^+$.

Example 47

*N*-hydroxy-*N*-benzo[b]thien-2-ylmethyl-*N'*-ethyl urea

The desired material was prepared according to the method of example 2, method a, except using ethyl isocyanate instead of trimethylsilyl isocyanate and using 2-benzo[b]thiophene carboxaldehyde instead of 2-acetylbenzothiophene. ($R_1$ = $NHCH_2CH_3$, A = $CH_2$, X = S [2-isomer], Y = H).

Melting Point: 128-129 °C.

NMR (300 MHz, DMSO-$d_6$): 1.01 (t, $3H^3$, J = 7.5 Hz); 3.03-3.15 (m, $2H^2$); 4.74 (s, 2H), 7.02-7.10 (t, 1H, J = 6.0 Hz); 7.26-7.38 (m, 3H); 7.75-7.80 (m, 1H); 7.87-7.93 (m, 1H); 9.46 (s, 1H).

Mass spectrum (DCI-$NH_3$): 251 $(M+H)^+$, 268 $(M+NH_4)^+$, 180, 162, 147, 134.

Analysis ($C_{12}H_{14}N_2O_2S$): Calculated -- C: 57.58, H: 5.64, N: 11.19; Found C: 57.65, H: 5.73, N: 10.96.

Example 48

*N*-hydroxy-*N*-(1-benzo[b]thien-2-yl)-3-methylpropyl urea

The desired material was prepared according to the method of example 2, method a, except using 2-isobutyrylbenzo[b]thiophene instead of 2-acetylbenzo[b]thiophene. ($R_1$ = $NH_2$, A = $CH(CH_2CH(CH_3)_2)$, X = S [2-isomer], Y = H).

NMR (300 MHz, DMSO-$d_6$): 0.92 (m, 3H); 1.67 (m, 2H), 1.91 (m, 1H), 5.51 (m, 1H); 6.39 (s, 2H); 7.27 (s, 1H); 7.31 (m, 2H); 7.78 (m, 1H); 7.87 (m, 1H); 9.24 (s, 1H).

Mass spectrum (DCI-$NH_3$): 296 $(M+H)^+$, 279 $(M+NH_4)^+$, 261, 203, 147.

Example 49

*N*-hydroxy-*N*-benzo[b]fur-2-ylmethyl urea

The desired material was prepared according to the method of example 2, method a, except using 2-benzo[b]furan carboxaldehyde instead of 2-acerylbenzothiophene. ($R_1$ = $NH_2$, A = $CH_2$, X = O [2-isomer], Y = H).

Melting Point: 161-162 °C.

NMR (300 MHz, DMSO-$d_6$): 4.66 (s, 2H); 6.51 (s, 2H); 6.77 (s, 1H); 7.18-7.30 (m, 2H); 7.50-7.63 (m,

2H); 9.54 (s, 1H).

IR (KBr): 1575, 1630, 1670, 3490.

Mass spectrum (DCI-$NH_3$): 207 $(M+H)^+$, 224 $(M+NH_4)^+$, 191, 162, 146, 131.

Analysis ($C_{10}H_{10}N_2O_3$): Calculated -- C: 58.25, H: 4.89, N: 13.59; Found C: 57.86, H: 5.01, N: 13.52.

Example 50

*N*-hydroxy-*N*-benzo[b]fur-2-ylmethyl *N'*-methyl urea

The desired material was prepared according to the method of example 2, method a, except using 2-benzo[b]furan carboxaldehyde instead of 2-acetylbenzothiophene and using methyl isocyanate instead of trimethylsilyl isocyanate. ($R_1$ = $NHCH_3$, A = $CH_2$, X = O [2-isomer], Y = H).

Melting Point: 126-128 ° C.

NMR (300 MHz, DMSO-$d_6$): 2.63 (d, 3H, 4.5 Hz); 4.65 (s, 2H); 6.75 (s, 1H); 7.05 (q, 1H, 4.5 Hz); 7.18-7.30 (m, 2H); 7.50-7.61 (m, 2H); 9.48 (s, 1H).

IR (KBr): 1538, 1660, 3450, 3530.

Mass spectrum (DCI-$NH_3$): 221 $(M+H)^+$, 238 $(M+NH_4)^+$, 203, 146, 131.

Analysis ($C_{11}H_{12}N_2O_3$): Calculated -- C: 59.99, H: 5.49, N: 12.72; Found C: 59.76, H: 5.34, N: 12.66.

Example 51

*N*-hydroxy-*N*-[1-(6-benzyloxybenzo[b]fur-2-yl)ethyl] urea

The desired material was prepared according to the method of example 22, except using 4-benzyloxysalicylaldehyde instead of 5-chlorosalicylaldehyde. ($R_1$ = $NH_2$, A = $CH_2$, X = O [2-isomer], Y = $C_6H_5CH_2O$).

Melting Point: 153-155 ° C

NMR (300 MHz, DMSO-$d_6$): 1.42 (d, 3, J = 6.6); 5.13 (s, 2); 5.42 (q, 1, J = 6.6); 6.43 (br s, 2); 6.61 (s, 1); 6.91 (dd, 1, J = 2.2, J = 8.5); 7.19 (d, 1, J = 2.2); 7.27-7.50 (m, 6); 9.13 (s, 1).

Mass spectrum (DCI-$NH_3$): 327 $(M+H)^+$, 344 $(M+NH_4)^+$, 251.

Analysis ($C_{18}H_{18}N_2O_4$): Calculated -- C: 66.24, H: 5.56, N: 8.59; Found C: 65.71, H: 5.46, N: 8.19.

Example 52

*N*-hydroxy-*N*-[1-(6-benzyloxybenzo[b]fur-2-yl)ethyl]*N'*-methyl urea

The desired material was prepared according to the method of example 22, except using 4-benzyloxysalicylaldehyde instead of 5-chlorosalicylaldehyde and using methyl isocyanate instead of trimethylsilyl isocyanate. ($R_1$ = $NHCH_3$, A = $CH_2$, X = O [2-isomer], Y = $C_6H_5CH_2O$).

Melting Point: 172.5-174 ° C.

NMR (300 MHz, DMSO-$d_6$): 1.43 (d, 3, J = 7.0); 2.63 (d, 3, J = 7.0); 5.14 (s, 2); 5.40 (br q, 1, J = 7.0); 6.62 (m, 1); 6.91 (dd, 1, J = 2.2, J = 8.5); 6.97 (br q, 1, J-7.0); 7.20 (d, 1, J = 2.2); 7.30-7.50 (m, 6); 9.06 (s, 1).

Mass spectrum (DCI-$NH_3$): 341 $(M+H)^+$, 358 $(M+NH_4)^+$, 251.

Analysis ($C_{19}H_{20}N_2O_4$): Calculated -- C: 67.04, H: 5.92, N: 8.23; Found C: 66.87, H: 5.86, N: 8.14.

Example 53

*N*-hydroxy-*N*-(1-indol-2-yl)ethyl) urea

The desired material was prepared according to the method of example 38, except using trimethylsilyl isocyanate instead of methylisocyanate. ($R_1$ = $NH_2$, A = $CHCH_3$, X = NH [2-isomer], Y = H).

Melting Point: 159-160 ° C

NMR (300 MHz, DMSO-$d_6$): 1.48 (d, 3H, J = 7 Hz); 5.46 (q, 1H, J = 7 Hz); 6.28 (s, 1H); 6.45 (br s, 2H); 6.93 (m, 1H); 7.03 (m, 1H); 7.33 (m, 1H); 7.43 (m, 1H); 8.05 (s, 1H); 10.75 (br s, 1H).

Mass spectrum (DCI-$NH_3$): 220 $(M+H)^+$, 237 $(M+NH_4)^+$, 144.

Examples 54-59 are prepared in a manner generally analogous to methods of example 2.

Example 54

N-hydroxy-N-[1-(3-hydroxybenzo[b]thien-2-yl)ethyl] urea

($R_1$ = $NH_2$, A = CHCH3, X = S [2-isomer] , Y = 3-OH).

Example 55

N-hydroxy-N-[1-(5-trifluoromethylbenzo[b]thien-2-yl)ethyl] urea

($R_1$ = $NH_2$, A = $CHCH_3$, X = S [2-isomer] , Y = 5-$CF_3$).

Example 56

N-hydroxy-N-[1-(7-methoxybenzo[b]thien-2-yl)ethyl] urea

($R_1$ = $NH_2$, A = $CHCH_3$, X = S [2-isomer] , Y = 7-$OCH_3$).

Example 57

N-hydroxy-N-[1-(5-phenylbenzo[b]thien-2-yl)ethyl] urea

($R_1$ = $NH_2$, A = $CHCH_3$, X = S [2-isomer] , Y = 5-$C_6H_5$).

Example 58

N-hydroxy-N-[1-(5-benzyloxy-benzo[b]thien-2-yl)ethyl] urea

($R_1$ = $NH_2$, A = $CHCH_3$, X = S [2-isomer], Y = 5-$C_6H_5CH_2O$).

Example 59

N-hydroxy-N-[1-(2-benzo[b]thien-2-yl)propyl] urea

($R_1$ = $NH_2$, A = $CHCH_2CH_3$, X = S [2-isomer], Y = H).
Examples 60-66 are prepared in a manner generally analogous to methods of example 1.

Example 60

N-hydroxy-N-(1-benzo[b]thien-2-ylethyl) propionamide

($R_1$ = $CH_3CH_2$, A = $CHCH_3$, X = S [2-isomer], Y = H).

Example 61

N-hydroxy-N-(1-benzo[b]thien-2-ylethyl) propenamide

($R_1$ = $CH_2$ = CH, A = CHCH3, X = S [2-isomer], Y = H).

Example 62

N-hydroxy-N-indol-2-ylmethyl acetamide

($R_1$ = $CH_3$, A = $CH_2$, X = NH[2-isomer], Y = H).

Example 63

*N*-hydroxy-*N*-(1-benzo[b]thien-3-ylethyl) acetamide

($R_1$ = $CH_3$, A = $CHCH_3$, X = S [3-isomer], Y = H).

Example 64

*N*-hydroxy-*N*-[1-(5-fluorobenzo[b]fur-2-yl)ethyl] acetamide

($R_1$ = $CH_3$, A = $CHCH_3$, X = O [2-isomer], Y = 5-F).

Example 65

*N*-hydroxy-*N*-(1-(5-phenoxybenzo[b]fur-2-yl)ethyl) acetamide

($R_1$ = $CH_3$, A = $CHCH_3$, X = O [2-isomer], Y = 5-$C_6H_5O$).

Example 66

*N*-hydroxy-*N*-[1-(5-(4-fluorophenyl)methyl)benzo[b]thien-2-yl)ethyl acetamide

($R_1$ = $CH_3$, A = $CHCH_3$, X = O [2-isomer], Y = 5-(4-$FC_6H_5$)$CH_2$).

Example 67

*N*-hydroxy-*N*-(1-benzo[b]thien-2-ylethyl) urea potassium salt

The desired material is prepared as described in example 19 except potassium bis(trimethylsilyl) amide is used instead of sodium bis(trimethylsilyl) amide. ($R_1$ = $NH_2$, A = $CHCH_3$, X = S [2-isomer], Y = H, M = K).

Example 68

*N*-hydroxy-*N*-(1-benzo[b]thien-2-ylethyl) acetamide ammonium salt

The desired material is prepared as described in example 19 except ammonia is used instead of sodium bis(trimethylsilyl) amide. ($R_1$ = $CH_3$, A = $CHCH_3$, X = S [2-isomer], Y = H, M = $NH_4$).

Example 69

*N*-hydroxy-*N*-(1-benzo[b]thien-2-ylethyl) acetamide methyl ammonium salt

The material prepared as in example 1 is dissolved in tetrahydrofuran and one equivalent of triethylamine is added. The solvent is removed *in vacuo* to yield the desired product. $R_1$ = $CH_3$, A = $CHCH_3$, X = S [2-isomer], Y = H, M = $NH(C_2H_5)_3$.

Example 70

*N*-hydroxy-*N*-(1-benzo[b]thien-2-ylethyl) acetamide tetraethyl ammonium salt

The material prepared as in example 1 is dissolved in tetrahydrofuran and one equivalent of tetraethylammonium hydroxide is added. The solvent is removed *in vacuo* to yield the desired product. $R_1$ = $CH_3$, A = $CHCH_3$, X = S [2-isomer], Y = H, M = $N(C_2H_5)_4$.

Example 71

*N*-butyryloxy-*N*-(1-benzo[b]thien-2-ylethyl) urea

The material prepared as in example 2 and 1.1 equivalent of triethylamine are dissolved in tetrahydrofuran and 1 equivalent of butryryl chloride is added. Ether is added and the material is washed with 2N HCl, dried with $MgSO_4$ and then evaporated *in vacuo* to yield the desired product. ($R_1 = NH_2$, $A = CHCH_3$, X = S [2-isomer], Y = H, $M = COC_3H_7$).

Example 72

*N*-benzoyloxy-*N*-(1-benzo[b]thienylethyl) urea

The material prepared as in example 2 and 1.1 equivalent of triethylamine are dissolved in tetrahydrofuran and 1 equivalent of benzoyl chloride is added. Ether is added and the material is washed with 2N HCl, dried with $MgSO_4$ and then evaporated *in vacuo* to yield the desired product. ($R_1 = NH_2$, $A = CHCH_3$, X = S [2-isomer], Y = H, $M = COC_6H_5$).

Example 73

Lipoxygenase $IC_{50}$ Determination

Assays to determine 5-lipoxygenase inhibitory activity were performed in 200 µL incubations containing the 20,000*xg* supernatant from $6x10^4$ homogenized RBL-1 cells, 2% DMSO vehicle and various concentrations of the test compound. Reactions were initiated by addition of radiolabelled arachidonic acid and terminated by acidification and ether extraction. Reaction products were separated from nonconverted substrate by thin layer chromatography and measured by liquid scintillation spectroscopy. All treatments were evaluated in triplicate incubations. Inhibition of 5-lipoxygenase activity was computed by comparison of the quantity of products formed in the treatment incubations to the mean product formation in vehicle control groups (n = 8). $IC_{50}$ values and 95% confidence limits were computed by linear regression analysis of percentage inhibition versus log inhibitor concentration plots. Inhibitory potencies for representative examples of compounds of this invention are listed in Table 1.

**Table 1.** *In vitro* 5-lipoxygenase inhibitory potency of compounds of this invention.

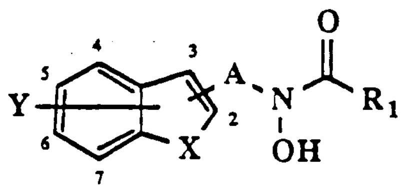

| Ex | $R_1$ | A | X | Y | Attached* | $IC_{50}$(μM) |
|---|---|---|---|---|---|---|
| 1 | $CH_3$ | $CHCH_3$ | S | H | 2 | 1.1 |
| 2 | $NH_2$ | $CHCH_3$ | S | H | 2 | 0.65 |
| 3 | $NHCH_3$ | $CHCH_3$ | S | H | 2 | 0.65 |
| 4 | $N(CH_3)_2$ | $CHCH_3$ | S | H | 2 | 0.54 |
| 5 | $CH_3$ | $CH_2$ | S | H | 2 | 1.6 |
| 6 | $NH_2$ | $CH_2$ | S | H | 2 | 1.9 |
| 7 | $N(CH_3)_2$ | $CH_2$ | S | H | 2 | 1.1 |
| 8 | $CH_3$ | $CHCH_3$ | S | H | 3 | 1.4 |
| 9 | $NH_2$ | $CHCH_3$ | S | H | 3 | 1.3 |
| 10 | $NH_2$ | $CHCH_3$ | S | 3-$CH_3$ | 2 | 0.54 |
| 11 | $NH_2$ | $CHCH_3$ | S | 5-$(CH_3)_3C$ | 2 | 0.71 |
| 12 | $CH_3$ | $CHCH_3$ | $SO_2$ | H | 2 | 21 |
| 14 | $CH_3$ | $CHCH_3$ | O | H | 2 | 1.9 |
| 15 | $NH_2$ | $CHCH_3$ | O | H | 2 | 2.7 |
| 16 | $CH_3$ | $CHCH_3$ | $NCH_3$ | H | 3 | 2.6 |
| 17 | $NH_2$ | $CHCH_3$ | $NCH_3$ | H | 3 | 2.8 |
| 18 | $NHCH_3$ | $CHCH_3$ | $NCH_3$ | H | 3 | 3.7 |
| 20 | H | $CHCH_3$ | S | H | 2 | 3.0 |
| 21 | $(CH_3)_2CH$ | $CHCH_3$ | S | H | 2 | 0.38 |
| 22 | $NH_2$ | $CHCH_3$ | O | 5-$CH_3O$ | 2 | 1.7 |
| 23 | $NH_2$ | $CHCH_3$ | O | 5-Cl | 2 | 0.81 |
| 24 | $NH_2$ | $CHCH_3$ | $NCH_3$ | H | 2 | 1.6 |
| 25 | $NH_2$ | $CHCH_3$ | S | H | 2 Z=S | 0.19 |
| 26 | $NH_2$ | $CHCH_3$ | S | 3-$CH_3O$ | 2 | 0.66 |
| 28 | $NH_2$ | $CHCH_3$ | S | 4,7-$(CH_3)_2$ | 2 | 0.40 |
| 29 | $NH_2$ | $CHCH_3$ | S | 5-F | 2 | 0.50 |
| 30 | $NH_2$ | $CH_2CHCH_3$ | S | H | 2 | 0.46 |
| 31 | $CH_3$ | $CH{=}CHCH_2$ | S | H | 2 | 0.27 |
| 32 | $NH_2$ | $CH{=}CHCH_2$ | S | H | 2 | 0.35 |
| 33 | $NH_2$ | $CHCH_3$ | O | 5,7-$Cl_2$ | 2 | 0.36 |
| 34 | $NH_2$ | $CHCH_3$ | O | 7-$CH_3O$ | 2 | 1.9 |
| 36 | $NH_2$ | $CHCH_3$ | O | 7-$CH_3CH_2O$ | 2 | 3.5 |
| 37 | $NH_2$ | $CHCH_3$ | NH | H | 2 | 2.9 |
| 38 | $NH_2$ | $CHCH_3$ | NH | 5-Cl | 2 | 1.0 |
| 39 | $NH_2$ | $CHCH_3$ | $NCOCH_3$ | H | 2 | 4.0 |
| 40 | $NH_2$ | $CHCH_3$ | $NSO_2CH_3$ | H | 2 | 3.0 |
| 41 | $NHCH_3$ | $CH_2$ | S | H | 7 | 7.2 |

| | | | | | | | |
|---|---|---|---|---|---|---|---|
| 42 | $NH_2$ | $CHCH_3$ | O | 2,3-dihydro | 2 | | 8.5 |
| 43 | NHOH | $CHCH_3$ | S | H | 2 | | 0.51 |
| 44 | $NHCH_2CH_3$ | $CHCH_3$ | S | H | 2 | | 1.8 |
| 45 | $NHCH_3$ | $CHCH_3$ | S | H | 2 | Z=S | 0.19 |
| 46 | $NHCH_3$ | $CH_2$ | S | H | 2 | | 1.9 |
| 47 | $NHCH_2CH_3$ | $CH_2$ | S | H | 2 | | 1.0 |
| 48 | $NH_2$ | $CH(CH_2CH(CH_3)_2)$ | S | H | 2 | | 0.2 |
| 49 | $NH_2$ | $CH_2$ | O | H | 2 | | 8.2 |
| 50 | $NHCH_3$ | $CH_2$ | O | H | 2 | | 2.3 |
| 51 | $NH_2$ | $CHCH_3$ | O | $5\text{-}C_6H_5CH_2O$ | 2 | | 0.25 |
| 52 | $NHCH_3$ | $CHCH_3$ | O | $5\text{-}C_6H_5CH_2O$ | 2 | | 0.20 |
| 53 | $NH_2$ | $CHCH_3$ | NH | H | 2 | | 2.9 |

*Position at which side chain is attached to the heterocyclic ring system.

Example 74

Rat Peritoneal Anaphylaxis Model

Assays to determine the ability of compounds to prevent the synthesis of 5-lipoxygenase products *in vivo* after oral administration were performed as follows: Fasted male Sprague-Dawley derived rats (SASCO Inc., Oregon WI) were passively sensitized by i.p. injection of rabbit anti-bovine serum albumin (anti-BSA) in phosphate buffered saline (PBS), pH 7.1. Three hours after sensitization, the rats were injected i.p. with BSA (4 mg) in PBS (5 mL) containing 30 mM 1-cysteine. This initiates the synthesis of leukotrienes in the peritoneal cavity. Test compounds suspended in 0.2% methylcellulose or vehicle controls were administered by gavage 1 hour prior to the antigen challenge. Typically 6-8 rats were included in both the control and treatment groups.

The rats were sacrificed 15 minutes after challenge, the peritoneal cavity opened and the fluid contents collected with a plastic trocar. The cavities were rinsed with cold PBS, pH 7.4, (5 mL) containing gelatin (5 mg), sodium azide (5 mg), EDTA (18.8 mg) and 1-cysteine (30 mM). These fluids were transferred to ice-old methanol, incubated for about 20 minutes, vortexed and then centrifuged at 1000*xg* for 15 minutes. Fluid volumes were recorded and the samples stored frozen until radioimmunoassay (New England Nuclear, Boston, MA) for $LTC_4$ equivalents was conducted.

Analysis of variance followed by Duncan's multiple range test was used to determine the statistical significance of treatment effects. Percent inhibition values were determined by comparing the treatment values to the mean of the control group. Inhibitory potencies for representative examples of compounds of this invention are listed in Table 2. The results of this assay demonstrate that compounds of this invention prevent the in vivo biosynthesis of the products of 5-lipoxygenase action on arachidonic acid

**Claims**

Table 2

| *In vivo* 5-lipoxygenase inhibitory potency of compounds of this invention in the rat peritoneal anaphylaxis model after oral administration. | | | |
|---|---|---|---|
| Example | % Inhibition at 200 $\mu$ mole/kg PO | Example | % Inhibition at 200 $\mu$ mole/kg PO |
| 1 | 67 | 28 | 67 |
| 2 | 98 | 29 | 89 |
| 3 | 79 | 30 | 79 |
| 4 | 78 | 31 | 30 |
| 5 | 79 | 32 | 35 |
| 9 | 26 | 33 | 84 |
| 10 | 69 | 34 | 69 |
| 13 | 53 | 35 | 85 |
| 14 | 76 | 45 | 33 |
| 15 | 75% at 100 $\mu$mole/kg | 46 | 77 |
| 16 | 81 | 47 | 73 |
| 17 | 59 | 48 | 66 |
| 18 | 47 | 50 | 81 |
| 19 | 61% at 150 $\mu$mole/kg | 51 | 46 |
| 20 | <40% | 52 | <40% |
| 22 | 91 | 53 | 76 |
| 23 | 60% at 100 $\mu$mole/kg | | |
| 25 | 72 | | |
| 26 | 56 | | |

**Claims for the following Contracting States : BE, CH, DE, FR, GB, IT, LI, NL, SE**

**1.** A compound of the formula:

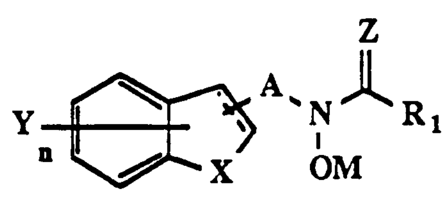

wherein $R_1$ is (1) hydrogen, (2) $C_1$ to $C_4$ alkyl, (3) $C_2$ to $C_4$ alkenyl, or (4) $NR_2R_3$, wherein $R_2$ and $R_3$ are independently selected from (1) hydrogen, (2) $C_1$ to $C_4$ alkyl and (3) hydroxyl, but $R_2$ and $R_3$ are not simultaneously hydroxyl;

wherein X is oxygen, sulfur, $SO_2$, or $NR_4$, wherein $R_4$ is (1) hydrogen, (2) $C_1$ to $C_6$ alkyl, (3) $C_1$ to $C_6$ alkoyl, (4) aroyl, or (5) alkylsulfonyl;

A may be attached to any available position on either ring and is selected from $C_1$ to $C_6$ alkylene and $C_2$ to $C_6$ alkenylene;

n is 1-5;

Y may be attached to any available position on either ring and is selected independently at each

occurrence from (1) hydrogen, (2) halogen, (3) hydroxy, (4) cyano, (5) halosubstituted alkyl, (6) $C_1$ to $C_{12}$ alkyl, (7) $C_2$ to $C_{12}$ alkenyl, (8) $C_1$ to $C_{12}$ alkoxy, (9) $C_3$ to $C_8$ cycloalkyl, (10) $C_1$ to $C_8$ thioalkyl, (11) aryl, (12) aryloxy, (13) aroyl, (14) arylalkyl in which the alkyl portion is of from 1 to 12 carbon atoms, (15) arylalkenyl in which the alkyl portion is of from 2 to 12 carbon atoms, (16) arylalkoxy in which the alkoxy portion is of from 1 to 12 carbon atoms, (17) arylthioalkoxy in which the alkoxy portion is of from 1 to 12 carbon atoms, and substituted derivatives of (18) aryl, (19) aryloxy, (20) aroyl, (21) arylalkyl in which the alkyl is of from 1 to 12 carbon atoms, (22) arylalkenyl in which the alkenyl portion is of from 2 to 12 carbon atoms, (23) arylalkoxy in which the alkoxy portion is of from 1 to 12 carbon atoms, or (24) arylthioalkoxy in which the alkoxy portion is of from 1 to 12 carbon atoms, wherein substituents are selected from halo, nitro, cyano, $C_1$ to $C_{12}$ alkyl, $C_1$ to $C_{12}$ alkoxy, and $C_1$ to $C_{12}$ halosubstituted alkyl;

Z is oxygen or sulfur;

M is hydrogen, a pharmaceutically acceptable cation, aroyl, or $C_1$ to $C_{12}$ alkoyl;

wherein aryl refers to both carbocyclic and heterocyclic aromatic radicals;

and wherein the dotted line within the five membered ring represents either a single or double bond.

**2.** A compound of claim 1 having the formula:

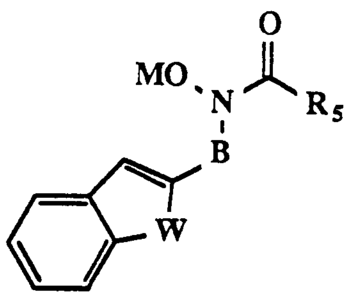

wherein $R_5$ is $C_1$ or $C_2$ alkyl, or $NR_6R_7$ where $R_6$ and $R_7$ are independently selected from hydrogen and $C_1$ or $C_2$ alkyl; B is $Ch_2$ or $CHCH_3$; W is oxygen or sulfur; and M is hydrogen, a pharmaceutically acceptable cation, aroyl, or $C_1$ to $C_{12}$ alkoyl.

**3.** The compound of claim 1 having the formula:

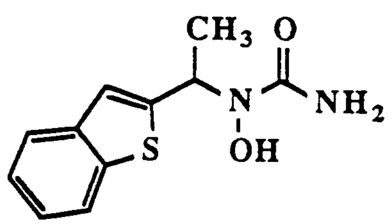

**4.** The compound of claim 1 having the formula:

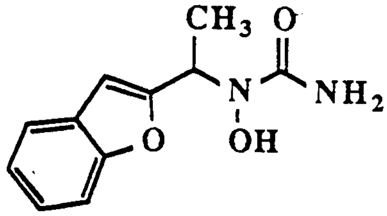

5. Use of a compound of claim 1 for preparing a drug useful for inhibiting 5- and/or 12-lipoxygenase activity in a mammal in need of such treatment.

6. Use of claim 5 wherein the compound has the formula:

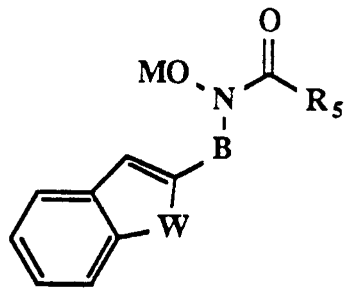

wherein $R_5$ is $C_1$ or $C_2$ alkyl, or $NR_6R_7$ where $R_6$ and $R_7$ are independently selected from hydrogen and $C_1$ or $C_2$ alkyl; B is $CH_2$ or $CHCH_3$; W is oxygen or sulfur; and M is hydrogen, a pharmaceutically acceptable cation, aroyl, or $C_1$ to $C_{12}$ alkoyl.

7. Use of claim 5 wherein the compound has the formula:

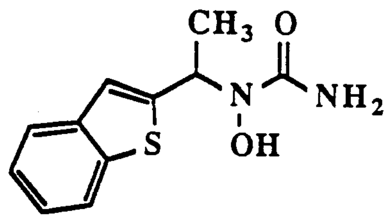

8. Use of claim 5 wherein the compound has the formula:

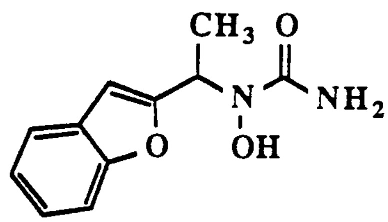

9. Use of a compound of claim 1 for preparing a drug useful for treating asthma, rheumatoid arthritis, gout, psoriasis, allergic rhinitis, adult respiratory distress syndrome, Crohn's disease, endotoxin shock, inflammatory bowel disease and/or ischemia induced myocardial or brain injury .

10. A pharmaceutical composition for inhibiting 5- and/or 12-lipoxygenase, comprising a pharmaceutical carrier and a therapeutically effective amount of a compound of claim 1.

**Claims for the following Contracting States : ES, GR**

**1.** A process for preparing a compound of the formula:

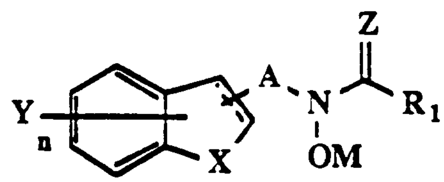

wherein $R_1$ is (1) hydrogen, (2) $C_1$ to $C_4$ alkyl, (3) $C_2$ to $C_4$ alkenyl, or (4) $NR_2R_3$, wherein $R_2$ and $R_3$ are independently selected from (1) hydrogen, (2) $C_1$ to $C_4$ alkyl and (3) hydroxyl, but $R_2$ and $R_3$ are not simultaneously hydroxyl;

X is oxygen, sulfur, $SO_2$, or $NR_4$, wherein $R_4$ is (1) hydrogen, (2) $C_1$ to $C_6$ alkyl, (3) $C_1$ to $C_6$ alkoyl, (4) aroyl, or (5) alkylsulfonyl;

A may be attached to any available position on either ring and is selected from $C_1$ to $C_6$ alkylene and $C_2$ to $C_6$ alkenylene;

n is 1-5;

Y may be attached to any available position on either ring and is selected independently at each occurrence from (1) hydrogen, (2) halogen, (3) hydroxy, (4) cyano, (5) halosubstituted alkyl, (6) $C_1$ to $C_{12}$ alkyl, (7) $C_2$ to $C_{12}$ alkenyl, (8) $C_1$ to $C_{12}$ alkoxy, (9) $C_3$ to $C_8$ cycloalkyl, (10) $C_1$ to $C_8$ thioalkyl, (11) aryl, (12) aryloxy, (13) aroyl, (14) arylalkyl in which the alkyl portion is of from 1 to 12 carbon atoms, (15) arylalkenyl in which the alkyl portion is of from 2 to 12 carbon atoms, (16) arylalkoxy in which the alkoxy portion is of from 1 to 12 carbon atoms, (17) arylthioalkoxy in which the alkoxy portion is of from 1 to 12 carbon atoms, and substituted derivatives of (18) aryl, (19) aryloxy, (20) aroyl, (21) arylalkyl in which the alkyl is of from 1 to 12 carbon atoms, (22) arylalkenyl in which the alkenyl portion is of from 2 to 12 carbon atoms, (23) arylalkoxy in which the alkoxy portion is of from 1 to 12 carbon atoms, or (24) arylthioalkoxy in which the alkoxy portion is of from 1 to 12 carbon atoms, wherein substituents are selected from halo, nitro, cyano, $C_1$ to $C_{12}$ alkyl, $C_1$ to $C_{12}$ alkoxy, and $C_1$ to $C_{12}$ halosubstituted alkyl;

Z is oxygen or sulfur;

M is hydrogen, a pharmaceutically acceptable cation, aroyl, or $C_1$ to $C_{12}$ alkoyl;

wherein aryl refers to both carbocyclic and heterocyclic aromatic radicals;

said process comprising the steps of alternatively:

a) when Z is oxygen and $R_1$ is hydrogen, $C_1$ to $C_4$ alkyl, $C_2$ to $C_4$ alkenyl, or $N(C_1$ to $C_4$ alkyl$)_2$

1) reacting a compound of the structure

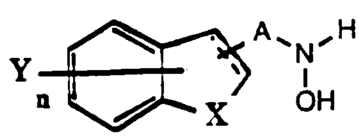

where A, n, X and Y are as defined above, with an acid chloride of the structure $R_1COCl$ where $R_1$ is as defined above in the presence of an acid scavenger to produce a compound of the structure

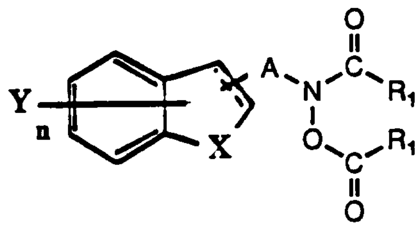

where A, n, X, Y and $R_1$ are as defined above, and thereafter

2) hydrolyzing the product of step (1) in the presence of lithium hydroxide to produce a compound of the structure

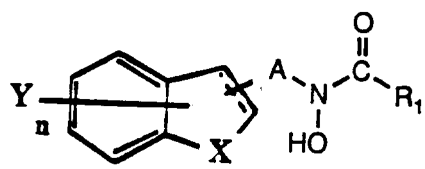

where A, n, X, Y and $R_1$ are as defined above; or

b) when Z is sulfur and $R_1$ is hydrogen, $C_1$ to $C_4$ alkyl, $C_2$ to $C_4$ alkenyl, or $N(C_1$ to $C_4$ alkyl$)_2$

1) reacting a compound of the structure

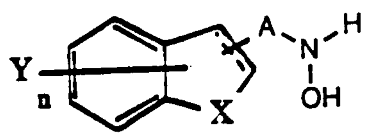

where A, n, X and Y are as defined above, with an acid chloride of the structure $R_1CSCl$ where $R_1$ is as defined above in the presence of an acid scavenger to produce a compound of the structure

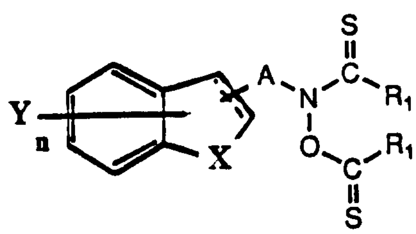

where A, n, X, Y and $R_1$ are as defined above, and thereafter

2) hydrolyzing the product of step (1) in the presence of lithium hydroxide to produce a compound of the structure

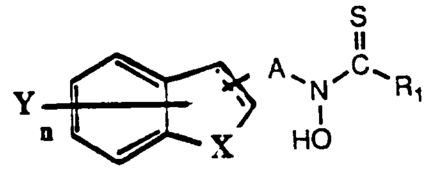

where A, n, X, Y and $R_1$ are as defined above; or

c) when Z is oxygen and $R_1$ is $NR_2R_3$ where $R_2$ and $R_3$ are hydrogen, or $R_2$ is alkyl and $R_3$ is hydrogen,

1) reacting a compound of the structure

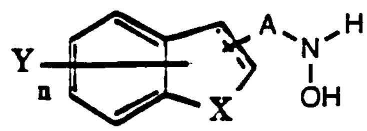

where A, n, X and Y are as defined above, with trimethylsilylisocyanate or with $R_2$-N=C=O where $R_2$ is alkyl followed by hydrolysis or alternatively with sodium or potassium cyanate in acidic solution to produce a compound of the structure

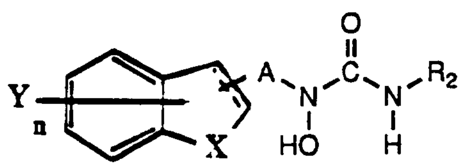

where A, n, X and Y are as defined above and $R_2$ is hydrogen or alkyl; or

d) when Z is sulfur and $R_1$ is $NR_2R_3$ where $R_2$ and $R_3$ are hydrogen, or $R_2$ is alkyl and $R_3$ is hydrogen,

1) reacting a compound of the structure

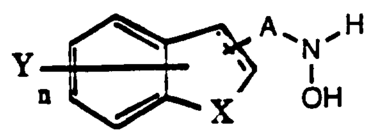

where Y, n, A and X are as defined above, with trimethylsilylisothiocyanate or with $R_2$-N=C=S where $R_2$ is alkyl followed by hydrolysis or alternatively with sodium or potassium thiocyanate in acidic solution to produce a compound of the structure

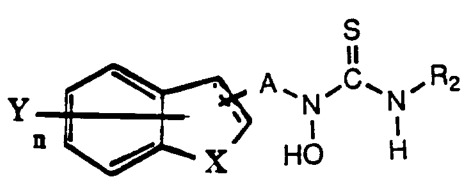

where A, n, X and Y are as defined above and $R_2$ is hydrogen or alkyl; or

e) when Z is oxygen and $R_1$ is $NR_2R_3$ where $R_2$ and $R_3$ are both hydrogen or one of $R_2$ and $R_3$ is hydrogen and the other is hydroxyl

1) reacting a compound of the structure

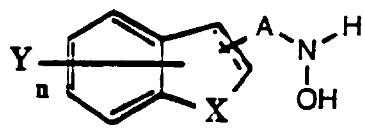

where A, n, X and Y are as defined above with phosgene to form a compound of the structure

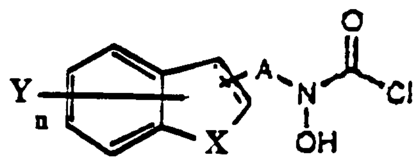

where A, n, X and Y are as defined above and

2) thereafter reacting the product of step (1) with ammonium hydroxide if both $R_2$ and $R_3$ are hydrogen or reacting the product of step (1) with hydroxylamine if one of $R_2$ and $R_3$ is hydrogen and the other is hydroxyl to produce a compound of the structure

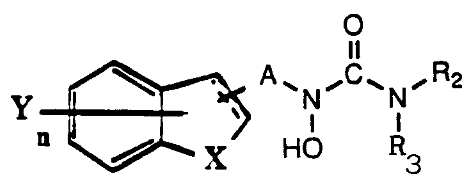

where A, n, X and Y are as defined above and $R_2$ and $R_3$ are both hydrogen or one of $R_2$ and $R_3$ is hydrogen and the other is hydroxyl.

f) when Z is sulfur and $R_1$ is $NR_2R_3$ where $R_2$ and $R_3$ are both hydrogen or one of $R_2$ and $R_3$ is hydrogen and the other is hydroxyl

1) reacting a compound of the structure

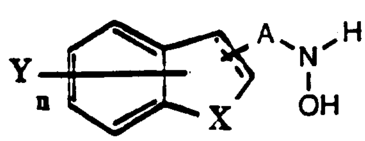

where A, n, X and Y are as defined above with thiophosgene to form a compound of the structure

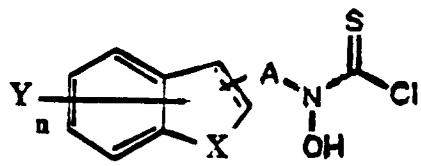

where A, n, X and Y are as defined above and
2) thereafter reacting the product of step (1) with ammonium hydroxide if both $R_2$ and $R_3$ are hydrogen or reacting the product of step (1) with hydroxylamine if one of $R_2$ and $R_3$ is hydrogen and the other is hydroxyl to produce a compound of the structure

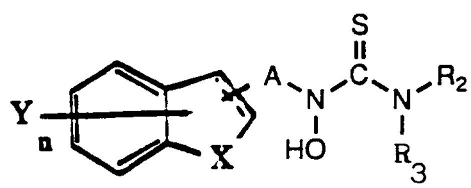

where A, n, X and Y are as defined above and $R_2$ and $R_3$ are both hydrogen or one of $R_2$ and $R_3$ is hydrogen and the other is hydroxyl.

**2.** A process as defined in Claim 1 wherein $R_1$ is $C_1$ or $C_2$ alkyl, or $NR_2R_3$ where $R_2$ and $R_3$ are independently selected from hydrogen and $C_1$ or $C_2$ alkyl; A is $CH_2$ or $CHCH_3$; X is oxygen or sulfur; and Y is oxygen.

**3.** A process as defined in Claim 2 wherein $R_1$ is $NH_2$, A is $CHCH_3$ and X is sulfur.

**4.** A process as defined in Claim 3 for preparing a compound of the structure

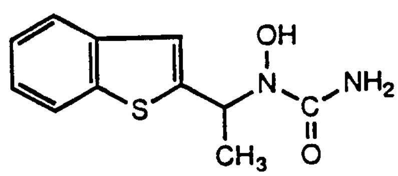

comprising the steps of
1) reacting a compound of the structure

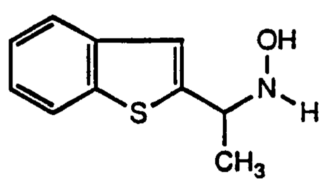

with trimethylsilylisocyanate followed by hydrolysis or alternatively with sodium or potassium cyanate in acidic solution to produce a compound of the structure

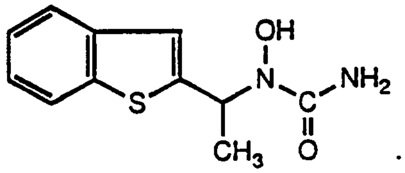

**5.** A process as defined in Claim 2 wherein $R_1$ is $NH_2$, A is $CHCH_3$ and X is oxygen.

**6.** A process as defined in Claim 5 for preparing a compound of the structure

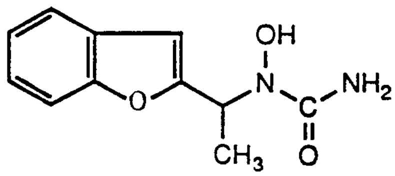

comprising the steps of

1) reacting a compound of the structure

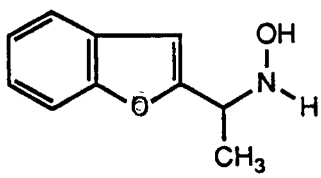

with trimethylsilylisocyanate followed by hydrolysis or alternatively with sodium or potassium cyanate in acidic solution to produce a compound of the structure

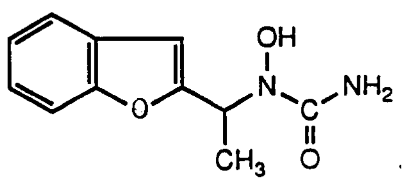

**Patentansprüche**
**Patentansprüche für folgende Vertragsstaaten : BE, CH, DE, FR, GB, IT, LI, NL, SE**

1. Eine Verbindung mit der Formel:

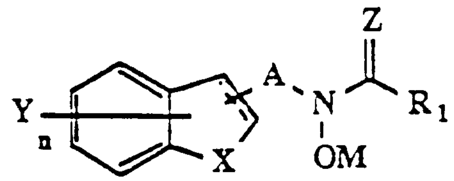

dadurch gekennzeichnet,

daß $R_1$ entweder (1) Wasserstoff, (2) ein $C_1$- bis $C_4$-Alkyl, (3) ein $C_2$- bis $C_4$-Alkenyl oder (4) $NR_2R_3$ ist, worin $R_2$ und $R_3$ unabhängig voneinander aus (1) Wasserstoff, (2) einem $C_1$- bis $C_4$-Alkyl oder (3) einer Hydroxylgruppe gewählt sind, jedoch nicht beide gleichzeitig einer Hydroxylgruppe zugehören;

daß X Sauerstoff, Schwefel, $SO_2$ oder $NR_4$ ist, worin $R_4$ (1) Wasserstoff, (2) ein $C_1$- bis $C_6$-Alkyl, (3) ein $C_1$- bis $C_6$-Alkoylrest, (4) ein Aroylrest oder (5) ein Alkylsulfonylrest ist;

daß A an jeder beliebigen verfügbaren Stelle in beiden Ringen gebunden sein kann und entweder ein $C_1$- bis $C_6$-Alkylen oder ein $C_2$- bis $C_6$-Alkenylen ist;

daß n 1 bis 5 ist;

daß Y an jeder beliebigen verfügbaren Stelle in beiden Ringen gebunden sein kann und jedesmal unabhängig gewählt ist aus (1) Wasserstoff, (2) Halogen, (3) Hydroxylgruppe, (4) Cyanogruppe, (5) halogensubstituertem Alkyl, (6) $C_1$- bis $C_{12}$-Alkyl, (7) $C_2$- bis $C_{12}$-Alkenyl, (8) einem $C_1$- bis $C_{12}$-Alkoxylrest, (9) $C_3$- bis $C_8$-Cycloalkyl, (10) $C_1$- bis $C_8$ -Thioalkyl, (11) Aryl, (12) einem Aryloxyrest, (13) einem Aroylrest, (14) einem Arylalkylrest, worin der Alkylanteil aus 1 bis 12 Kohlenstoffatomen besteht, (15) einem Arylalkenylrest, worin der Alkylanteil aus 2 bis 12 Kohlenstoffatomen besteht, (16) einem Arylalkoxyrest, worin der Alkoxyanteil aus 1 bis 12 Kohlenstoffatomen besteht, (17) einem Arylthioalkoxyrest, worin der Alkoxyanteil aus 1 bis 12 Kohlenstoffatomen besteht, und substituierten Derivaten von (18) Aryl, (19) einem Aryloxyrest, (20) Aroyl, (21) einem Arylalkylrest, worin der Alkylanteil aus 1 bis 12 Kohlenstoffatomen besteht, (22) einem Arylalkenylrest, worin der Alkenylanteil aus 2 bis 12 Kohlenstoffatomen besteht, (23) einem Arylalkoxyrest, worin der Alkoxyanteil aus 1 bis 12 Kohlenstoffatomen besteht, oder (24) einem Arylthioalkoxyrest, worin der Alkoxyanteil aus 1 bis 12 Kohlenstoffatomen besteht, wobei die Substituenten aus Halogen, Nitrogruppe, Cyanogruppe, $C_1$- bis $C_6$-Alkyl, $C_1$- bis $C_{12}$-Alkoxyrest und halogensubstitiuiertem $C_1$- bis $C_{12}$-Alkyl auszuwählen sind;

daß Z Sauerstoff oder Schwefel ist;

daß M Wasserstoff, ein pharmazeutisch zulässiges Kation, ein Aroylrest oder ein $C_1$- bis $C_{12}$-Alkoylrest ist;

daß sich Aryl sowohl auf carbocyclische als auch auf heterocyclische aromatische Radikale bezieht;

und daß die punktierte Linie innerhalb des fünfgliedrigen Rings entweder eine Einfach- oder eine Doppelbindung darstellt.

2. Eine Verbindung nach Anspruch 1 mit der Formel:

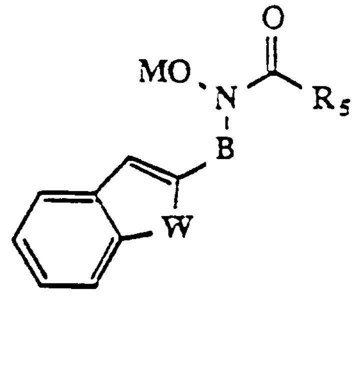

dadurch gekennzeichnet, daR $R_5$ ein $C_1$- oder $C_2$-Alkyl oder $NR_6R_7$ ist, worin $R_6$ und $R_7$ unabhängig voneinander Wasserstoff oder ein $C_1$- oder $C_2$-Alkyl sind; B ist $CH_2$ oder $CHCH_3$; W ist Sauerstoff oder Schwefel und M ist Wasserstoff, ein pharmazeutisch zulässiges Kation, ein Aroylrest oder ein $C_1$- bis $C_{12}$-Alkoylrest.

**3.** Die Verbindung nach Anspruch 1 mit der Formel:

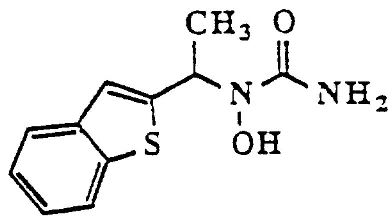

**4.** Die Verbindung nach Anspruch 1 mit der Formel:

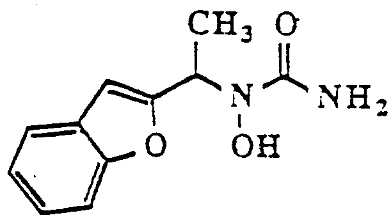

**5.** Verwendung einer Verbindung nach Anspruch 1 für die Herstellung eines Arzneimittels, das für die Inhibition der Aktivität von 5- und/oder 12-Lipoxygenase in einem Säugetier, das eine solche Behandlung benötigt, hilfreich ist.

**6.** Verwendung nach Anspruch 5, dadurch gekennzeichnet, daß die Verbindung folgende Formel hat:

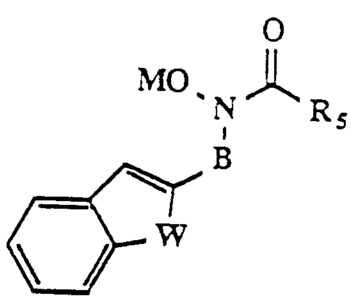

worin $R_5$ ein $C_1$- oder $C_2$-Alkyl oder $NR_6R_7$ ist, worin $R_6$ und $R_7$ unabhängig voneinander Wasserstoff oder ein $C_1$- oder $C_2$-Alkyl sind; B ist $CH_2$ oder $CHCH_3$; W ist Sauerstoff oder Schwefel und M ist Wasserstoff, ein pharmazeutisch zulässiges Kation, ein Aroylrest oder ein $C_1$- bis $C_{12}$-Alkoylrest.

7. Verwendung nach Anspruch 5, dadurch gekennzeichnet, daß die Verbindung folgende Formel hat:

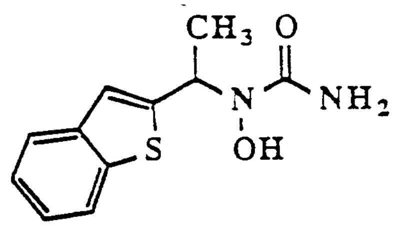

8. Verwendung nach Anspruch 5, dadurch gekennzeichnet, daß die Verbindung folgende Formel hat:

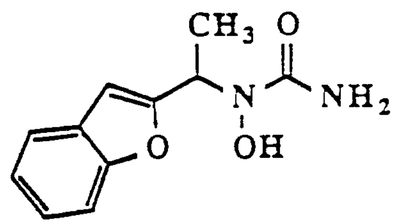

9. Verwendung einer Verbindung nach Anspruch 1 für die Herstellung eines Arzneimittels für die Behandlung von Asthma, rheumatoide Arthritis, Gicht, Schuppenflechte, allergischer Nasenschleimhautentzündung, Atemnotsyndrom bei Erwachsenen, Crohnischer Krankheit, endotoxinem Schock, entzündlicher Darmkrankheit und/oder von durch unzureichende Durchblutung herbeigeführten Herz- oder Gehirnschäden.

10. Eine pharmazeutische Verbindung für die Inhibition von 5- und/oder 12-Lipoxygenase, bestehend aus einer pharmazeutischen Trägersubstanz und einer therapeutisch wirksamen Menge einer Verbindung von Anspruch 1.

**Patentansprüche für folgende Vertragsstaaten : ES, GR**

1. Ein Verfahren zur Herstellung einer Verbindung mit der Formel:

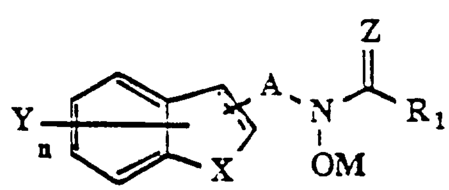

dadurch gekennzeichnet,

daß $R_1$ entweder (1) Wasserstoff, (2) ein $C_1$- bis $C_4$-Alkyl, (3) ein $C_2$- bis $C_4$-Alkenyl oder (4) $NR_2R_3$ ist, worin $R_2$ und $R_3$ unabhängig voneinander aus (1) Wasserstoff, (2) einem $C_1$- bis $C_4$-Alkyl oder (3) einer Hydroxylgruppe gewählt sind, jedoch nicht beide gleichzeitig einer Hydroxylgruppe zugehören;

daß X gleich Sauerstoff, Schwefel, $SO_2$ oder $NR_4$, worin $R_4$ (1) Wasserstoff, (2) ein $C_1$- bis $C_6$-Alkyl, (3) ein $C_1$- bis $C_6$-Alkoylrest, (4) ein Aroylrest oder (5) ein Alkylsulfonylrest ist;

daß A an jeder beliebigen verfügbaren Stelle in beiden Ringen gebunden sein kann und entweder ein $C_1$- bis $C_6$-Alkylen oder ein $C_2$- bis $C_6$-Alkenylen ist;

daß n 1 bis 5 ist;

daß Y an jeder beliebigen verfügbaren Stelle in beiden Ringen gebunden sein kann und jedesmal unabhängig gewählt ist aus (1) Wasserstoff, (2) Halogen, (3) Hydroxylgruppe, (4) Cyanogruppe, (5)

halogensubstituertem Alkyl, (6) $C_1$- bis $C_{12}$-Alkyl, (7) $C_2$- bis $C_{12}$-Alkenyl, (8) einem $C_1$- bis $C_{12}$-Alkoxylrest, (9) $C_3$- bis $C_8$-Cycloalkyl, (10) $C_1$- bis $C_8$ -Thioalkyl, (11) Aryl, (12) einem Aryloxyrest, (13) einem Aroylrest, (14) einem Arylalkylrest, worin der Alkylanteil aus 1 bis 12 Kohlenstoffatomen besteht, (15) einem Arylalkenylrest, worin der Alkylanteil aus 2 bis 12 Kohlenstoffatomen besteht, (16) einem Arylalkoxyrest, worin der Alkoxyanteil aus 1 bis 12 Kohlenstoffatomen besteht, (17) einem Arylthioalkoxyrest, worin der Alkoxyanteil aus 1 bis 12 Kohlenstoffatomen besteht, und substituierten Derivaten von (18) Aryl, (19) einem Aryloxyrest, (20) Aroyl, (21) einem Arylalkylrest, worin der Alkylanteil aus 1 bis 12 Kohlenstoffatomen besteht, (22) einem Arylalkenylrest, worin der Alkenylanteil aus 2 bis 12 Kohlenstoffatomen besteht, (23) einem Arylalkoxyrest, worin der Alkoxyanteil aus 1 bis 12 Kohlenstoffatomen besteht, oder (24) einem Arylthioalkoxyrest, worin der Alkoxyanteil aus 1 bis 12 Kohlenstoffatomen besteht, wobei die Substituenten aus Halogen, Nitrogruppe, Cyanogruppe, $C_1$- bis $C_6$-Alkyl, $C_1$- bis $C_{12}$-Alkoxyrest und halogensubstitiuiertem $C_1$- bis $C_{12}$-Alkyl auszuwählen sind;

daß Z Sauerstoff oder Schwefel ist;

daß M Wasserstoff, ein pharmazeutisch zulässiges Kation, ein Aroylrest oder ein $C_1$- bis $C_{12}$-Alkoylrest ist;

daß sich Aryl sowohl auf carbbcyclische als auch auf heterocyclische aromatische Radikale bezieht;

wobei das Verfahren alternativ die folgenden Schritte aufweist:

a) wenn Z Sauerstoff ist und $R_1$ Wasserstoff, ein $C_1$- bis $C_4$-Alkyl, ein $C_2$- bis $C_4$-Alkenyl oder $N(C_1$-bis $C_4$-Alkyl$)_2$,

1) Reaktion einer Verbindung mit der Struktur

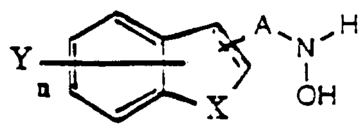

wobei A, n, X und Y wie oben definiert sind, mit einem Säurechlorid mit der Struktur $R_1COCl$ worin $R_1$ wie oben definiert ist, in Gegenwart eines sauren Desoxiationsmittel zur Herstellung einer Verbindung mit der Struktur

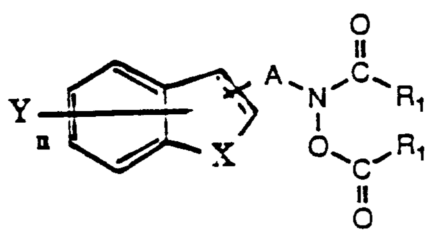

wobei A, n, X, Y und $R_1$ wie oben definiert sind, und anschließender

2) Hydrolyse des Produkt von Schritt (1) in Gegenwart von Lithiumhydroxid zur Herstellung einer Verbindung mit der Struktur

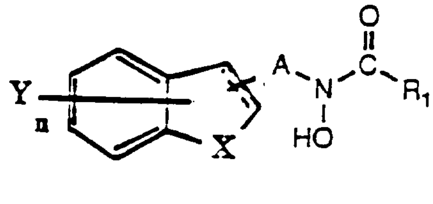

wobei A, n, X, Y und $R_1$ wie oben definiert sind; oder

b) wenn Z Schwefel ist und $R_1$ Wasserstoff, ein $C_1$- bis $C_4$-Alkyl, ein $C_2$- bis $C_4$-Alkenyl oder $N(C_1$-bis $C_4$-Alkyl$)_2$,

1) Reaktion einer Verbindung mit der Struktur

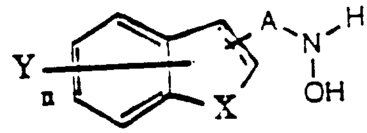

wobei A, n, X und Y wie oben definiert sind, mit einem Säurechlorid mit der Struktur $R_1$CSCl, worin $R_1$ wie oben definiert ist,in Gegenwart eines sauren Desoxidationsmittel zur Herstellung einer Verbindung mit der Struktur

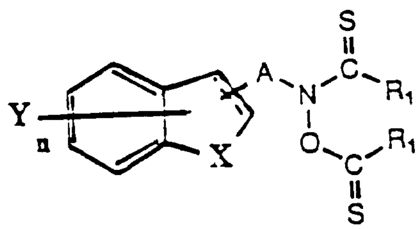

wobei A, n, X, Y und $R_1$ wie oben definiert sind, und anschließender

2) Hydrolyse des Produkt von Schritt (1) in Gegenwart von Lithiumhydroxid zur Herstellung einer Verbindung mit der Struktur

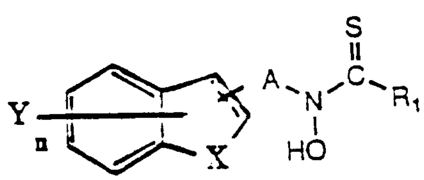

wobei A, n, X, Y und $R_1$ wie oben definiert sind; oder

c) wenn Z Sauerstoff ist und $R_1$ gleich $NR_2R_3$, worin $R_2$ und $R_3$ Wasserstoff oder $R_2$ ein Alkyl und $R_3$ Wasserstoff ist,

1) Reaktion einer Verbindung mit der Struktur

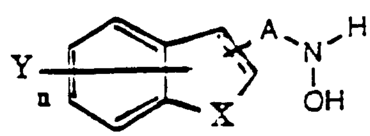

wobei A, n, X und Y wie oben definiert sind, mit Trimethylsilylisocyanat oder mit $R_2$-N=C=O, worin $R_2$ ein Alkyl ist, und nachfolgender Hydrolyse, oder alternativ mit Natrium- oder Kaliumcyanat in saurer Lösung für die Herstellung einer Verbindung mit der Struktur

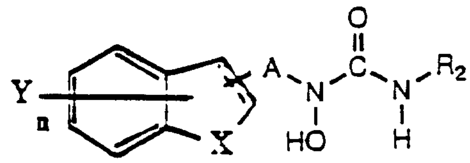

wobei A, n, X und Y wie oben definiert sind und $R_2$ Wasserstoff oder ein Alkyl ist; oder

d) wenn Z Schwefel ist und $R_1$ gleich $NR_2R_3$, worin $R_2$ und $R_3$ Wasserstoff oder $R_2$ ein Alkyl und $R_3$ Wasserstoff ist,

1) Reaktion einer Verbindung mit der Struktur

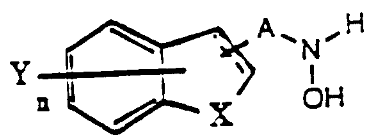

wobei Y, n, A und X wie oben definiert sind, mit Trimethylsilylisocyanat oder mit $R_2$-N=C=S, worin $R_2$ ein Alkyl ist, und nachfolgender Hydrolyse, oder alternativ mit Natrium- oder Kaliumthiocyanat in saurer Lösung für die Herstellung einer Verbindung mit der Struktur

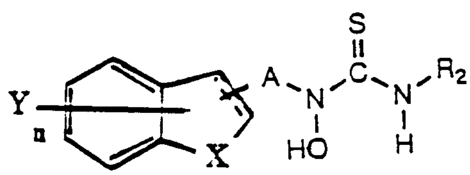

wobei A, n, X und Y wie oben definiert sind und $R_2$ Wasserstoff oder ein Alkyl ist; oder

e) wenn Z Sauerstoff ist und $R_1$ gleich $NR_2R_3$, worin $R_2$ und $R_3$ entweder beide Wasserstoff sind oder eins von beiden Wasserstoff und das andere eine Hydroxylgruppe ist

1) Reaktion einer Verbindung mit der Struktur

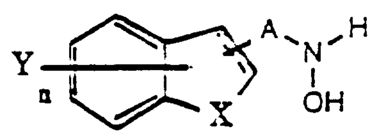

wobei A, n, X und Y wie oben definiert sind, mit Phosgen zur Herstellung einer Verbindung mit der Struktur

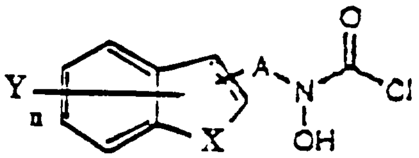

wobei A, n, X und Y wie oben definiert sind und

2) anschließender Reaktion des Produktes von Schritt (1) mit Ammoniumhydroxid, wenn sowohl $R_2$ als auch $R_3$ Wasserstoff ist, oder Reaktion des Produktes von Schritt (1) mit Hydroxylamin, wenn eins von $R_2$ und $R_3$ Wasserstoff und das andere eine Hydroxylgruppe ist, zur Herstellung einer Verbindung mit der Struktur

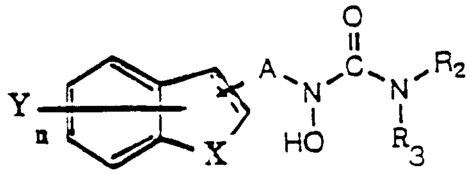

wobei A, n, X und Y wie oben definiert sind und $R_2$ und $R_3$ entweder beide Wasserstoff sind oder eins von beiden Wasserstoff und das andere eine Hydroxylgruppe ist.

f) wenn Z Schwefel ist und $R_1$ gleich $NR_2R_3$, worin $R_2$ und $R_3$ entweder beide Wasserstoff sind oder eins von beiden Wasserstoff und das andere eine Hydroxylgruppe ist

1) Reaktion einer Verbindung mit der Struktur

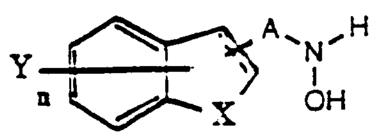

wobei A, n, X und Y wie oben definiert sind, mit Thiophosgen zur Herstellung einer Verbindung mit der Struktur

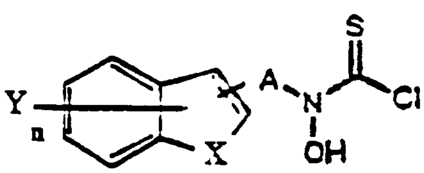

wobei A, n, X und Y wie oben definiert sind und

2) anschließender Reaktion des Produktes von Schritt (1) mit Ammoniumhydroxid, wenn sowohl $R_2$ als auch $R_3$ Wasserstoff ist, oder Reaktion des Produktes von Schritt (1) mit Hydroxylamin, wenn eins von $R_2$ und $R_3$ Wasserstoff und das andere eine Hydroxylgruppe ist, zur Herstellung einer Verbindung mit der Struktur

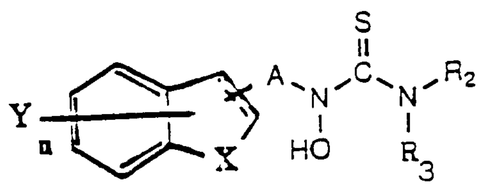

wobei A, n, X und Y wie oben definiert sind und $R_2$ und $R_3$ entweder beide Wasserstoff sind oder eins von beiden Wasserstoff und das andere eine Hydroxylgruppe ist.

**2.** Ein Verfahren nach Anspruch 1, dadurch gekennzeichnet, daß $R_1$ ein $C_1$- oder $C_2$-Alkyl ist oder $NR_2R_3$, worin $R_2$ und $R_3$ unabhängig voneinander Wasserstoff oder ein $C_1$- oder $C_2$-Alkyl sind; A ist $CH_2$ oder $CHCH_3$; X ist Sauerstoff oder Schwefel und Y ist Sauerstoff.

**3.** Ein Verfahren nach Anspruch 2, dadurch gekennzeichnet, daß $R_1$ gleich $NH_2$ ist, A gleich $CHCH_3$ und X Schwefel.

**4.** Ein Verfahren nach Anspruch 3 für die Herstellung einer Verbindung mit der Struktur

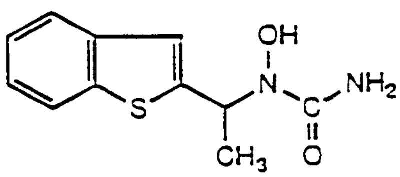

mit den Schritten

1) Reaktion einer Verbindung mit der Struktur

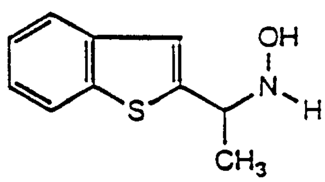

mit Trimethylsilylisocyanat mit nachfolgender Hydrolyse, oder alternativ dazu mit Natrium- oder Kaliumcyanat in saurer Lösung für die Herstellung einer Verbindung mit der Struktur

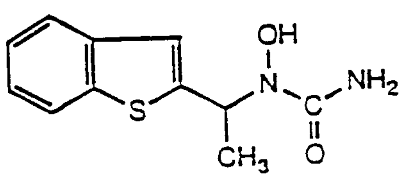

**5.** Ein Verfahren nach Anspruch 2, dadurch gekennzeichnet, daß $R_1$ gleich $NH_2$ ist, A gleich $CHCH_3$ und X Sauerstoff.

**6.** Ein Verfahren nach Anspruch 5 für die Herstellung einer Verbindung mit der Struktur

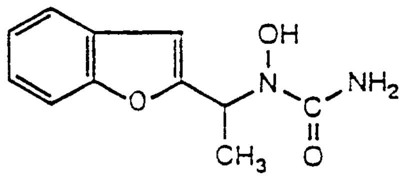

mit den Schritten

1) Reaktion einer Verbindung mit der Struktur

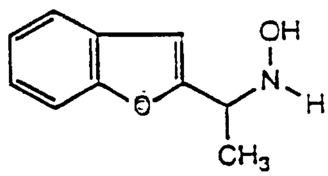

mit Trimethylsilylisocyanat mit nachfolgender Hydrolyse, oder alternativ dazu mit Natrium- oder Kaliumcyanat in saurer Lösung für die Herstellung einer Verbindung mit der Struktur

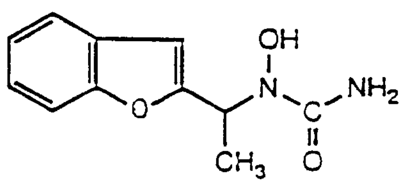

**Revendications**

**Revendications pour les Etats contractants suivants : BE, CH, DE, FR, GB, IT, LI, NL, SE**

**1.** Un composé de formule :

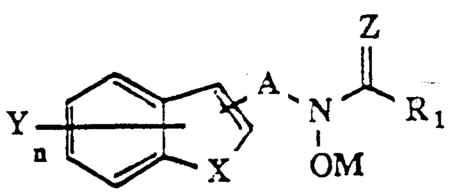

dans laquelle $R_1$ est (1) l'hydrogène, (2) un groupe $C_1$-$C_4$ alkyle, (3) un groupe $C_2$-$C_4$ alcényle, ou un bien (4) $NR_2R_3$, où $R_2$ et $R_3$ sont choisis indépendamment parmi (1) l'hydrogène, (2) un groupe $C_1$-$C_4$ alkyle et (3) un groupe hydroxyle, mais $R_2$ et $R_3$ ne sont pas simultanément un groupe hydroxyle ;
dans laquelle X est un atome d'oxygène, de souffle, un groupe $SO_2$ ou $NR_4$, où $R_4$ est (1) l'hydrogène, (2) un groupe $C_1$-$C_6$ alkyle, (3) un groupe $C_1$-$C_6$ alcoyle, (4) aroyle ou (5) alkylsulfonyle;
A peut être attaché dans une position disponible quelconque sur l'un ou l'autre noyau et est choisi parmi un groupe $C_1$-$C_6$ alkylène et $C_2$-$C_6$ alcénylène ;
n est égal à 1-5 ;
Y peut être attaché dans une position disponible quelconque sur l'un ou l'autre noyau et est choisi indépendamment chaque fois parmi (1) l'hydrogène, (2) un atome d'halogène, (3) un groupe hydroxy,

(4) cyano, (5) un groupe alkyle halosubstitué, (6) un groupe $C_1$-$C_{12}$ alkyle, (7) un groupe $C_2$-$C_{12}$ alcényle, (8) un groupe $C_1$-$C_{12}$ alcoxy, (9) un groupe $C_3$-$C_8$ cycloalkyle, (10) un groupe $C_1$-$C_8$ thioalkyle, (11) un groupe aryle, (12) un groupe aryloxy, (13) un groupe aroyle, (14) un groupe arylalkyle dans lequel la portion alkyle a de 1 à 12 atomes de carbone, (15) un groupe arylalcényle dans lequel la portion alkyle a de 2 à 12 atomes de carbone, (16) un groupe arylalcoxy dans lequel la portion alcoxy a de 1 à 12 atomes de carbone, (17) un groupe arylthioalcoxy dans lequel la portion alcoxy a de 1 à 12 atomes de carbone, et les dérivés substitués de (18) aryle, (19) aryloxy, (20) aroyle, (21) arylalkyle dans lequel alkyle a de 1 à 12 atomes de carbone, (22) arylalcényle dans lequel la portion alcényle a de 2 à 12 atomes de carbone, (23) arylalcoxy dans lequel la portion alcoxy a de 1 à 12 atomes de carbone, ou (24) arylthioalcoxy dis lequel la portion alcoxy a de 1 à 12 atomes de carbone, dans laquelle les substituants sont choisis parmi les suivants : un groupe halo, nitro, cyano, $C_1$-$C_{12}$ alkyle, $C_1$-$C_{12}$ alcoxy et $C_1$-$C_{12}$ alkyle substitué ;

Z est un atome d'oxygène ou de soufre ;

M est l'hydrogène, un cation pharmaceutiquement acceptable, un groupe aroyle ou $C_1$-$C_{12}$ alcoyle ;

dans laquelle aryle désire à la fois des radicaux aromatiques carbocycliques et hétérocycliques ;

et dans laquelle le trait discontinu à l'intérieur du noyau à cinq chaînons représente une liaison simple ou une double liaison.

**2.** Un composé selon la revendication 1 ayant la formule :

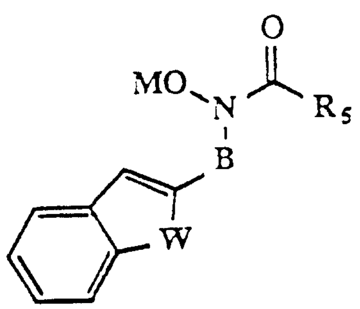

dans laquelle $R_5$ est un groupe $C_1$ ou $C_2$ alkyle, ou $NR_6R_7$ où $R_6$ et $R_7$ sont choisis indépendamment parmi l'hydrogène et le groupe $C_1$ ou $C_2$ alkyle ; B est $CH_2$ ou $CHCH_3$ ; W est un atome d'oxygène ou de soufre ; et M est l'hydrogène, un cation pharmaceutiquement acceptable, un groupe aroyle, ou $C_1$-$C_{12}$ alcoyle.

**3.** Le composé selon la revendication 1 ayant la formule :

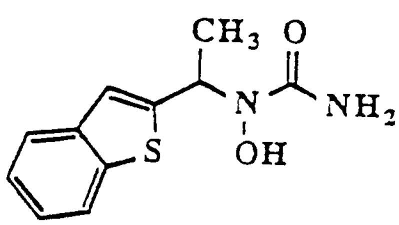

**4.** Le composé selon la revendication 1 ayant la formule :

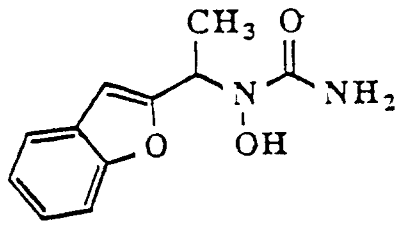

**5.** Utilisation d'un composé selon la revendication 1, pour la préparation d'un médicament utile pour inhiber l'activité de la 5- et/ou 12-lipoxygénase chez un mammifère ayant besoin d'un tel traitement.

**6.** Utilisation selon la revendication 5, selon laquelle le composé a la formule :

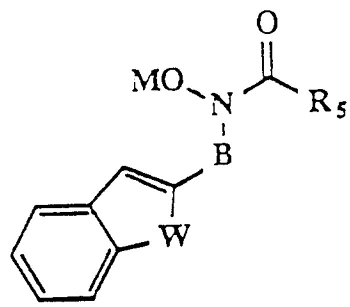

dans laquelle $R_5$ est un groupe $C_1$ ou $C_2$ alkyle, ou $NR_6R_7$ où $R_6$ et $R_7$ sont choisis indépendamment parmi l'hydrogène et un groupe $C_1$ ou $C_2$ alkyle ; B est $CH_2$ ou $CHCH_3$ ; W est un atome d'oxygène ou de soufre ; et M est l'hydrogène, un cation pharmaceutiquement acceptable, un groupe aroyle ou $C_1$-$C_{12}$ alcoyle.

**7.** Utilisation selon la revendication 5, selon laquelle le composé a la formule :

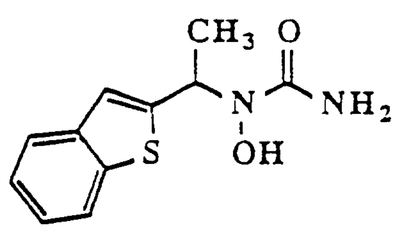

**8.** Utilisation selon la revendication 5, selon laquelle a la formule :

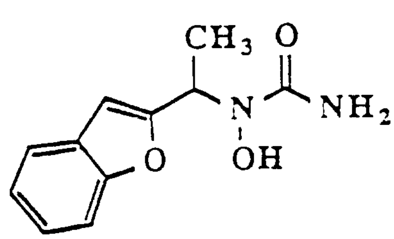

**9.** Utilisation d'un composé selon la revendication 1, pour la préparation d'un médicament utile pour le traitement de l'asthme, de l'arthrite rhumatismale, de la goutte, du psoriasis, de la rhinite allergique, du syndrome de détresse respiratoire chez l'adulte, de la maladie de Crohn, du choc d'endotoxine, des infections intestinales inflammatoires et/ou des lésions du cerveau ou du myocarde induites par l'ischémie.

**10.** Une composition pharmaceutique pour inhiber la 5- et/ou la 12-lipoxygénase comprenant un support pharmaceutique et une quantité thérapeutiquement efficace d'un composé selon la revendication 1.

**Revendications pour les Etats contractants suivants : ES, GR**

**1.** Un procédé de préparation d'un composé de formule :

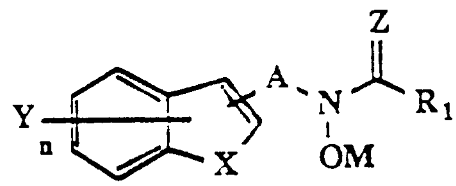

dans laquelle $R_1$ est (1) l'hydrogène, (2) un groupe $C_1$-$C_4$ alkyle, (3) un groupe $C_2$-$C_4$ alcényle, ou un bien (4) $NR_2R_3$, où $R_2$ et $R_3$ sont choisis indépendamment parmi (1) l'hydrogène, (2) un groupe $C_1$-$C_4$ alkyle et (3) un groupe hydroxyle, mais $R_2$ et $R_3$ ne sont pas simultanément un groupe hydroxyle ;
dans laquelle X est un atome d'oxygène, de soufre, un groupe $SO_2$ ou $NR_4$, où $R_4$ est (1) l'hydrogène, (2) un groupe $C_1$-$C_6$ alkyle, (3) un groupe $C_1$-$C_6$ alcoyle, (4) aroyle ou (5) alkylsulfonyle ;
A peut être attaché dans une position disponible quelconque sur l'un ou l'autre noyau et est choisi parmi un groupe $C_1$-$C_6$ alkylène et $C_2$-$C_6$ alcénylène ;
n est égal à 1-5 ;
Y peut être attaché dans une position disponible quelconque sur l'un ou l'autre noyau et est choisi indépendamment chaque fois parmi (1) l'hydrogène, (2) un atome d'halogène, (3) un groupe hydroxy, (4) cyano, (5) un groupe alkyle halosubstitué, (6) un groupe $C_1$-$C_{12}$ alkyle, (7) un groupe $C_2$-$C_{12}$ alcényle, (8) un groupe $C_1$-$C_{12}$ alcoxy, (9) un groupe $C_3$-$C_8$ cycloalkyle, (10) un groupe $C_1$-$C_8$ thioalkyle, (11) un groupe aryle, (12) un groupe aryloxy, (13) un groupe aroyle, (14) un groupe arylalkyle dans lequel la portion alkyle a de 1 à 12 atomes de carbone, (15) un groupe arylalcényle dans lequel la portion alkyle a de 2 à 12 atomes de carbone, (16) un groupe arylalcoxy dans lequel la portion alcoxy a de 1 à 12 atomes de carbone, (17) un groupe arylthioalcoxy dans lequel la portion alcoxy a de 1 à 12 atomes de carbone, et les dérivés substitués de (18) aryle, (19) aryloxy, (20) aroyle, (21) arylalkyle dans lequel alkyle a de 1 à 12 atomes de carbone, (22) arylalcényle dans lequel la portion alcényle a de 2 à 12 atomes de carbone, (23) arylalcoxy dans lequel la portion alcoxy a de 1 à 12 atomes de carbone, ou (24) arylthioalcoxy dans lequel la portion alcoxy a de 1 à 12 atomes de carbone, dans laquelle les substituants sont choisis parmi les suivants : un groupe halo, nitro, cyano, $C_1$-$C_{12}$ alkyle, $C_1$-$C_{12}$ alcoxy et $C_1$-$C_{12}$ alkyle substitué ;
Z est un atome d'oxygène ou de soufre ;
M est l'hydrogène, un cation pharmaceutiquement acceptable, un groupe aroyle ou $C_1$-$C_{12}$ alcoyle ;
dans laquelle aryle désigne à la fois des radicaux aromatiques carbocycliques et hétérocycliques ;
ledit procédé comprenant les étapes alternatives suivantes :

a) lorsque Z est l'oxygène et $R_1$ est l'hydrogène, un groupe $C_1$-$C_4$ alkyle, un groupe $C_2$-$C_4$ alcényle, ou un groupe $N(C_1$-$C_4$ alkyle$)_2$

1) la réaction d'un composé de structure :

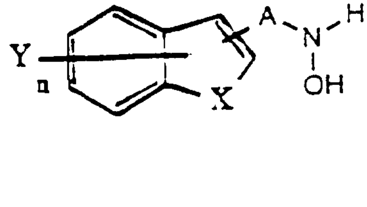

dans laquelle A, n, X et Y sont comme définis précédemment, avec un chlorure d'acide de structure $R_1COCl$ dans laquelle $R_1$ est comme défini précédemment en présence d'un agent de nettoyage d'acide pour produire un composé de structure :

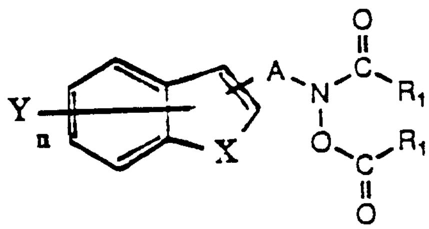

dans laquelle A, n, X, Y et $R_1$ sont comme définis précédemment et ensuite

2) l'hydrolyse du produit de l'étape (1) en présence de l'hydroxyde de lithium pour produire un composé de structure :

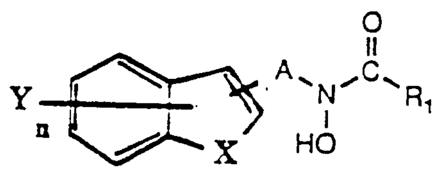

dans laquelle A, n, X, Y et $R_1$ sont comme définis précédemment ; ou bien

b) lorsque Z est un atome de soufre et $R_1$ est l'hydrogène, un groupe $C_1$-$C_4$ alkyle, un groupe $C_2$-$C_4$ alcényle, ou un groupe $N(C_1$-$C_4$ alkyle$)_2$

1) réaction des composés de structure :

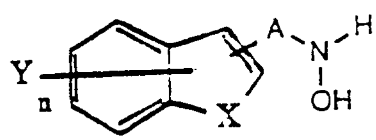

dans laquelle A, n, X et Y sont comme définis précédemment, avec un chlorure d'acide de structure $R_1CSCl$ dans laquelle $R_1$ est comme défini précédemment en présence d'un agent de nettoyage d'acide pour produire un composé de structure :

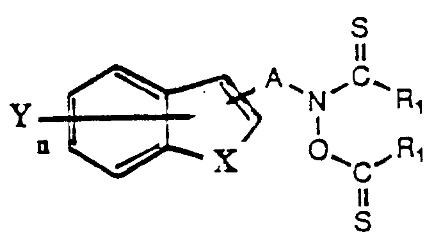

dans laquelle A, n, X, Y et $R_1$ sont comme définis précédemment, et ensuite

2) l'hydrolyse du produit de l'étape (1) en présence de l'hydroxyde de litnium pour produire un composé de structure :

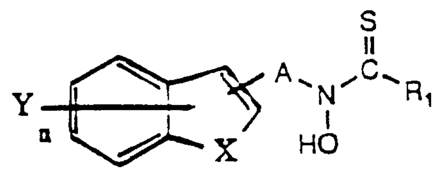

dans laquelle A, n, X, Y et $R_1$ sont comme définis précédemment ; ou bien

c) lorsque Z est un atome d'oxygène et $R_1$ est $NR_2R_3$ où $R_2$ et $R_3$ sont de l'hydrogène, ou bien $R_2$ est un groupe alkyle et $R_3$ est l'hydrogène,

1) la réaction d'un composé de structure :

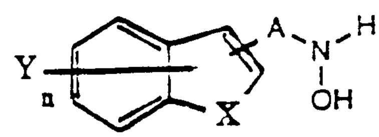

dans laquelle A, n, X et Y sont comme définis précédemment, avec l'isocyanate de triméthylsilyle ou avec le $R_2$-N=C=O où $R_2$ est un groupe alkyle suivie de l'hydrolyse ou altemativement avec le cyanate de sodium ou de potassium en solution acide pour produire un composé de structure :

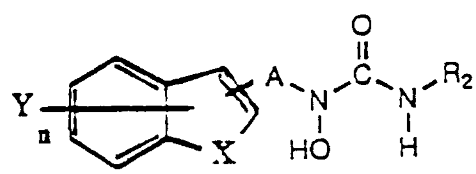

dans laquelle A, n, X et Y sont comme définis précédemment et $R_2$ est un atome d'hydrogène ou un groupe alkyle ; ou bien

d) lorsque Z est un atome de souffle et $R_1$ est $NR_2R_3$ où $R_2$ et $R_3$ sont de l'hydrogène ou bien $R_2$ est un groupe alkyle et $R_3$ est un atome d'hydrogène,

1) la réaction d'un composé de structure :

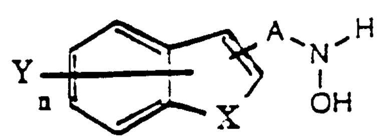

dans laquelle Y, n, A et X sont comme définis précédemment, avec l'isothiocyanate de triméthylsilyle ou avec $R_2$-N=C=S où $R_2$ est un groupe alkyle suivie de l'hydrolyse ou altemativement avec le thiocyanate de sodium ou de potassium en solution acide pour produire un composé de structure :

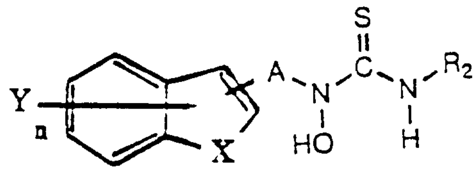

dans laquelle A, n, X et Y sont comme définis précédemment et $R_2$ est l'hydrogène ou un groupe alkyle ; ou bien

e) lorsque Z est un atome d'oxygène et $R_1$ est $NR_2R_3$ où $R_2$ et $R_3$ sont tous les deux de l'hydrogène ou l'un des deux $R_2$ et $R_3$ est l'hydrogène et l'autre est un groupe hydroxyle

1) la réaction d'un composé de structure :

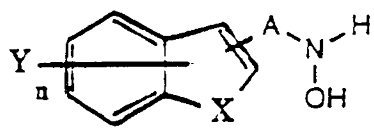

dans laquelle A, n, X et Y sont comme définis précédemment avec le phosgène pour former un composé de structure :

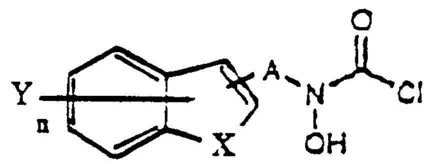

dans laquelle A, n, X et Y sont comme définis précédemment et

2) ensuite la réaction du produit de l'étape (1) avec l'hydroxyde d'ammonium lorsque les deux $R_2$ et $R_3$ sont de l'hydrogène ou la réaction du produit de l'étape (1) avec l'hydroxylamine lorsque l'un des $R_2$ et $R_3$ est de l'hydrogène et l'autre est un groupe hydroxyle pour produire un composé de structure :

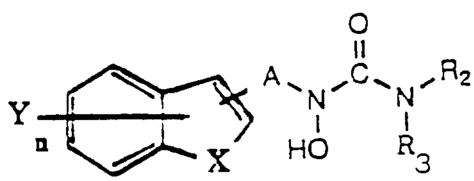

dans laquelle A, n, X et Y sont comme définis précédemment et $R_2$ et $R_3$ sont tous les deux de l'hydrogène ou bien l'un des $R_2$ et $R_3$ est l'hydrogène et l'autre est un groupe hydroxyle

f) lorsque Z est un atome de souffle et $R_1$ est $NR_2R_3$ où $R_2$ et $R_3$ sont tous les deux de l'hydrogène ou bien l'un des $R_2$ et $R_3$ est de l'hydrogène et l'autre est un groupe hydroxyle

1) la réaction d'un composé de structure :

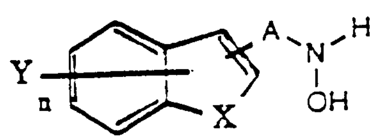

dans laquelle A, n, X et Y sont comme définis précédemment avec le thiophosgène pour former un composé de structure :

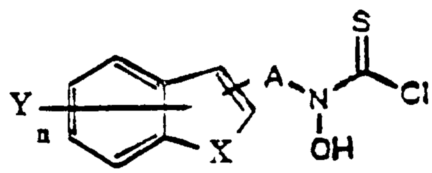

dans laquelle A, n, X et Y sont comme définis précédemment et

2) ensuite la réaction du produit de l'étape (1) avec l'hydroxyde d'ammonium lorsque $R_2$ et $R_3$ sont tous les deux de l'hydrogène ou la réaction du produit de l'étape (1) avec l'hydroxylamine lorsque l'un des $R_2$ et $R_3$ est l'hydrogène et l'autre est un groupe hydroxyle pour produùe un composé de structure :

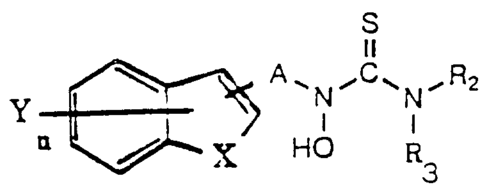

dans laquelle A, n, X et Y sont comme définis précédemment et $R_2$ et $R_3$ sont tous les deux de l'hydrogène ou bien l'un des $R_2$ et $R_3$ est de l'hydrogène et l'autre est un groupe hydroxyle.

**2.** Un procédé selon la revendication 1, selon laquelle $R_1$ est un groupe $C_1$ ou $C_2$ alkyle, ou $NR_2R_3$ où $R_2$ et $R_3$ sont choisis indépendemment parmi l'hydrogène et un groupe $C_1$ ou $C_2$ alkyle ; A est $CH_2$ ou $CHCH_3$; X est un atome d'oxygène ou de souffle ; et Y est un atome d'oxygène.

**3.** Un procédé selon la revendication 2, selon laquelle $R_1$ est $NH_2$, A est $CHCH_3$ et X est un atome de soufre.

**4.** Un procédé tel que défini dans la revendication 3, pour la préparation d'un composé de structure :

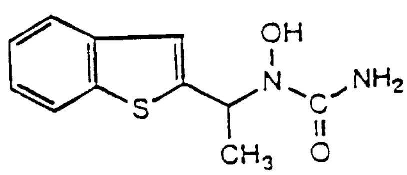

comprenant les étapes suivantes :

1) la réaction d'un composé de structure :

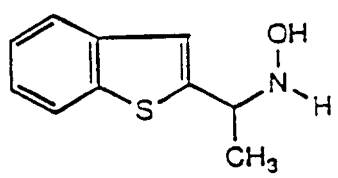

avec l'isocyanate de triméthylsilyle suivie de l'hydrolyse ou alternativement avec le cyanate de sodium ou de potassium en solution acide pour produire un composé de structure :

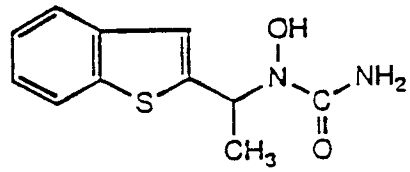

**5.** Un procédé selon la revendication 2, selon laquelle $R_1$ est $NH_2$, A est $CHCH_3$ et X est un atome d'oxygène.

**6.** Un procédé tel que défini dans la revendication 5, pour la préparation d'un composé de structure :

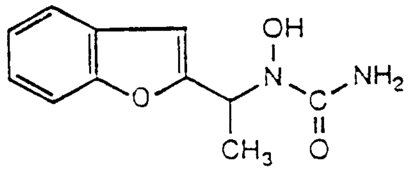

comprenant les étapes suivantes :

1) la réaction d'un composé de structure :

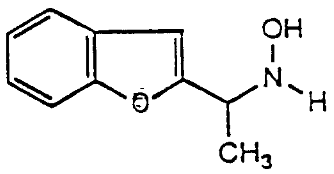

avec l'isocyanate de triméthylsilyle suivie de l'hydrolyse ou alternativement avec le cyanate de sodium ou de potassium en solution acide pour produire un composé de structure.